# EUROPEAN PATENT SPECIFICATION

(11) **EP 3 836 788 B1**
(45) Date of publication and mention of the grant of the patent: **16.09.2026**
(21) Application number: 20706371.0
(22) Date of filing: 14.02.2020
(51) Int. Cl.: A01N 57/16, A01N 63/60, A61K 39/00, C12N 15/00, A01P 1/00, A01P 3/00, A01P 5/00, A01P 7/02, A01P 7/04, A01P 13/02

(54) **COMPOSITION COMPRISING A MIXTURE OF DNA MOLECULES, USES THEREOF AS BIOLOGICAL INHIBITOR AND METHOD FOR PRODUCTION**
ZUSAMMENSETZUNG MIT EINER MISCHUNG AUS DNA-MOLEKÜLEN, IHRE VERWENDUNG ALS BIOLOGISCHER INHIBITOR UND VERFAHREN ZUR HERSTELLUNG
COMPOSITION COMPRENANT UN MÉLANGE DE MOLÉCULES D'ADN, SES UTILISATIONS COMME INHIBITEUR BIOLOGIQUE ET PROCÉDÉ DE PRODUCTION

(30) Priority: 14.02.2019 NL 2022581
(43) Date of publication of application: 23.06.2021
(73) Proprietor: Koppert B.V., 2651 BE Berkel en Rodenrijs (NL)
(72) Inventor: DIANO, Marcello Maria, 801046 Grazzanise (IT); LORITO, Matteo, 20010 Pellezzano (IT); MAZZOLENI, Stefano, 80121 Napoli (IT); MIKKELSEN, Harald Gijsbert, 3046 NJ Rotterdam (NL)
(74) Representative: Gitto, Serena
(86) International application number: PCT/NL2020/050086
(87) International publication number: WO 2020/167128

(56) References cited:
- WO-A1-2019/079656
- WO-A2-2014/020624
- STEFANO MAZZOLENI ET AL: "Inhibitory and toxic effects of extracellular self-DNA in litter: a mechanism for negative plant-soil feedbacks?", NEW PHYTOLOGIST, vol. 205, no. 3, 1 February 2015 (2015-02-01), GB, pages 1195 - 1210, XP055601496, ISSN: 0028-646X, DOI: 10.1111/nph.13121

## Description

### FIELD OF THE INVENTION

The present invention concerns methods and uses employing compositions comprising a mixture of DNA molecules suitable for inhibiting a target species, and methods for producing the compositions. Said compositions may find utility in amongst others the fields of human and/or veterinary medicine, pest and/or disease control in agriculture or in other fields where inhibition of one or more target species is beneficial.

### BACKGROUND

In the recent years knowledge has come available that fragmented DNA of a species is inhibitory for the species from which the DNA is derived and for phylogenetically similar species having a similar genome. The first publications disclosing this self-inhibitory effect of DNA fragments are the international patent application WO2014/020624 and its Italian priority application NA2012A000046. These documents focus mainly on the newly discovered effects of the self-inhibitory DNA fragments, which according to these publications are produced by sonication, heat treatment, or pyrolysis, of isolated total DNA from the species to be inhibited (or from a phylogenetically similar species) or by random DNA fragment synthesis starting from total DNA of the species (or from a phylogenetically similar species).

In order to employ the DNA fragments more efficiently for the inhibition of biological species, further methods for their production in effective form are desired. It has now surprisingly been found that DNA fragments of a species to be inhibited are effective after being incorporated in DNA not originating from the species to be inhibited. Based on this surprising finding, the inventors of the present invention have found that inhibitory DNA fragments of a species may also be effectively produced in a host species unrelated to the species to be inhibited. This opens new inventive possibilities for producing inhibitory DNA fragments.

### SUMMARY OF THE INVENTION

The invention according to a first aspect relates to a use of a composition as a product for inhibiting a species, the target species, said composition comprising a mixture of DNA molecules, having DNA sequences from different sources, the mixture comprising a number of first DNA sequences, preferably from chromosomal DNA sequences, of a species, the source species, and a number of second DNA sequences, from a species, the host species, used to replicate the source species DNA, said host species differing from the source species, wherein the source species is selected from the target species or is a species phylogenetically similar to the target species, wherein species are phylogenetically similar if they are from the same family, preferably from the same genus, and wherein the source species and the host species are phylogenetically distant by being selected from different taxonomic orders, such as by being selected from different classes, different phyla, different kingdoms, or different domains. In the composition the mixture of DNA molecules optionally comprises chimeric DNA molecules wherein source species DNA sequences are flanked on at least one end by non-source species DNA sequences selected from host species DNA sequences or from DNA sequences from an artificial DNA construct. Such a composition is beneficial in the inhibition of a target species selected as the source species, or selected from a species phylogenetically similar to the source species, in that the inhibitory DNA fragments can be produced in a host species that is phylogenetically distant from the target species.

A further aspect of the invention relates to a composition of the invention for use in medicine. In view of the fact that the composition of the invention may be used for inhibiting parasitic and/or pathogenic organisms, it has utility in human and/or veterinary medicine.

Yet a further aspect of the invention relates to the use of the composition according to the invention as a product for inhibiting a target species. This use may be a use in human and/or veterinary medicine or alternatively may be a use outside medicine. Examples of the use outside medicine may for example include uses in agriculture, where it may be beneficial to inhibit organisms that are parasitic and/or pathogenic and/or are pests for plants and/or are pests for life stock animals, such as cattle or poultry, or otherwise interfere with agricultural production, such as weeds. Alternatively, the composition could also be used as a preservative in for example products prone to microbial spoilage, such as food products, personal health care products, or other products comprising compounds suitable as microbial substrate.

The invention according to a further aspect relates to a method of inhibiting a species, the target species, comprising exposing said target species to a composition according to the invention. The method may be a non-therapeutic method or a therapeutic method, the latter being not part of the present invention.

According to a further aspect, the invention relates to a method of producing a composition according to the invention comprising:
- providing a source of DNA from the source species, optionally comprising fragmented source species DNA;
- providing cells of a number of species, the host species, differing from the source species;
- subjecting the host species cells with the source of DNA from the source species to conditions allowing the host species to take up source species DNA in a replicable form;
- isolating host species cells as a source of DNA inhibitory to the target species;
- optionally, isolating DNA inhibitory to the target species from the source species cells;
- optionally, fragmenting the DNA content of host species cells.

The method is beneficial in that DNA inhibitory for the target species can now be effectively produced outside the target species or a phylogenetically similar species. Self-inhibitory DNA can now in particular be produced in a species phylogenetically unrelated to the target species.

Still a further aspect of the invention relates to a method of producing an agricultural product. In the method, the composition of the invention is used for inhibiting organisms that are infections and/or parasitic and/or are pests for plants or for life stock animals, such as cattle or poultry otherwise interfere with agricultural production. By the inhibition of organisms that are infections and/or parasitic and/or are pests for plants or for life stock animals the agricultural production by such production organisms may be improved.

### DETAILED DESCRIPTION OF THE INVENTION

The composition to be used according to the invention comprises a mixture of DNA molecules having DNA sequences from different sources. The mixture of DNA molecules comprises a number of first DNA sequences, of a source species, and a number of second DNA sequences of a host species, differing from the source species. As the skilled person will understand the term "species" refers to an abstract concept and a species as such cannot be inhibited. Reference to a species should thus be construed as meaning individuals or organisms of the species, such as a plurality of individuals or organisms of the species, i.e. a population.

As the term mixture implies, the composition comprises differing DNA molecules. DNA molecules in the composition differ in sequence and preferably also in size. DNA sequences of the DNA molecules in the composition differ by being from different sources. In particular a first set of DNA molecules has sequences, of a first species, the source species, and a second set of DNA molecules has sequences of a second species, the host species, differing from the source species. In addition, the number of source species DNA sequences also differ. Furthermore, the number of host species DNA sequences also differs. Thus, there is variation also within the population of source species DNA sequences and within the population of host species DNA sequences.

In the composition, source species DNA sequences may be incorporated, preferably as partial sequences (fragments) of chromosomal DNA, in a DNA molecule of the natural genome of the host species, such as a chromosome or natural plasmid, from the natural genome of host species cells, or may be incorporated in an artificial DNA construct introduced into host species cells, thus forming chimeric DNA. Alternatively, source species DNA sequences in the composition have been disruptively liberated, for example by fragmentation, from such chimeric DNA. The DNA molecules in the composition may thus comprise one or more of whole (intact) natural chromosomes of a species, whole (intact) natural extra-chromosomal DNA, such as natural plasmids, of a species, whole (intact) artificial DNA constructs, such as selected from plasmids, cosmids, fosmids or artificial chromosomes, fragmented natural chromosomes of a species, fragmented natural extra-chromosomal DNA, such as natural plasmids, of a species, or fragmented artificial constructs, such as selected from plasmids, cosmids, formids or artificial chromosomes. In the composition of the invention, such DNA molecules may also be present in combinations. It will be clear for the skilled person that and how these alternative DNA molecules can differ in sequence and size.

The DNA molecules comprising the source species DNA sequences and the DNA molecules comprising host species DNA sequences preferably are DNA fragments. The skilled person will understand that the term "fragmented DNA" and related terms such as "DNA fragments" refers to a partial strand (or partial sequence) of a larger DNA molecule. DNA fragments may be obtained by DNA fragmentation (breaking down of DNA molecules) or by synthesis of DNA starting from a polynucleotide template. A DNA fragment is thus a DNA molecule having a partial sequence of a larger DNA molecule. In the context of the present invention, inhibitory DNA fragments preferably are obtained by DNA fragmentation. With the context of the present invention the term "comprising" and related terms, "such as comprises", include "consists of".

DNA fragments may be generated by any means suitable, in particular by techniques for random DNA fragment generation. WO2014/020624 discloses a number of suitable techniques for generating self-inhibitory DNA fragments. Amongst others, sonication, heat treatment, or pyrolysis, of isolated DNA from the source species are disclosed. Alternatively random DNA fragment synthesis starting from DNA of the source species may also be used for the random generation of DNA fragments. These and other techniques for generating DNA fragments are known to the skilled person and based on common general knowledge available they can be employed. Reference may further be made to Mann and Krull (Mann and Krull 2004. Biosensors and Bioelectronics 20:945-955), Tong et al, (Tong et al, 2006. Nat. Protoc, 1(2):729-748), Fan et al (Fan et al, (2008). Nucleic Acid Research, 36(19): e125) and Standard protocols DNA shearing for Bioruptor^{®} Pico (available via the diagenode website www.diagenode.com. Alternatively, endonucleases, such as DNAse I, endonuclease V or restriction enzymes may be used for fragmenting source species DNA.

DNA fragmentation is also frequently used in (next generation) DNA sequencing procedures. The skilled person may also draw from the body of knowledge developed in connection to DNA fragmentation in this context.

In addition, DNA synthesis methods that synthesize fractions of genomic sequences may also be used for obtaining DNA fragments. For example PCR techniques such as random PCR techniques may be suitable. Also cDNA molecules obtained by reverse transcription of mRNA may be considered to be DNA fragments within the context of the present invention as such cDNA molecules represent a partial sequence of a larger DNA molecule. Synthesis of suitable cDNA molecules is within the ambit of knowledge of the skilled person.

Furthermore, processes such as (biological) digestion, decay and similar processes working on biological materials, such as cells, containing DNA may also result in fragmentation of the DNA contained in the biological material. Thus it is not required that the DNA molecules comprising the source species DNA sequences are DNA fragments as DNA fragments can also be liberated from larger molecules by such processes.

The selected technique for DNA fragment generation, preferably is a technique suitable for random DNA fragment generation. Random DNA fragments should be understood to mean DNA fragments that are randomly generated. That is DNA fragments generated without a predetermined pattern.

From investigations relating to DNA fragmentation in the context of (next generation) DNA sequencing, knowledge has emerged that certain techniques for random DNA fragmentation, such as (partial) restriction enzyme digestion and sonication may have a degree of bias for certain areas and/or sequences of a genome. As the skilled person will understand from the inhibition of self-DNA as disclosed in WO2014/020624, this bias is not relevant for the inhibitory action of randomly generated fragments of self-DNA.

In the composition of the present invention the DNA molecules preferably are provided as double stranded DNA molecules (dsDNA). This does not necessarily mean that the DNA molecules perform their biological inhibitory function in a double stranded state. Without wishing to be bound by this theory, it appears plausible that for double stranded DNA molecules single stranded DNA sequences resulting from (partially) unwound dsDNA are involved in the inhibitory actions. DNA molecules in double stranded form thus may provide inhibitory action via mechanisms of (partial) DNA unwinding.

The composition may comprise DNA molecules having source species DNA sequences with a size ranging from about 50 bases (50 bp) to about 2 Mb (2*10⁶ bp), such as 50 to 3000 bp, 100 to 1500 bp, or 100 to 2000 bp, 100 to 2500 bp, or alternatively 0.3-15 kb, such as 1-10 kb, such as 5-10 kb, or alternatively 15-60 kb, such as 15-40, 25-40 kb, 24-or alternatively 40-340 kb, such as 50-200 kb, such as 120-300 kb, such as 200 - 300 kb, or alternatively 250 kb-2Mb, such as 400 kb-1500 kb or 400 kb -1000 kb, or alternatively 0.3 kb - 2 Mb, such as 1 kb - 2 Mb, such as 10 kb - 2 Mb, such as 100 kb - 2Mb, such as 360 kb-1 Mb. Dependent on whether the source species DNA sequences are free fragmented DNA sequences or whether they are incorporated in larger DNA molecules comprising non-source species DNA sequences, their size may vary. Free fragmented DNA sequences preferably will have sizes more in the direction of the lower ranges. Source species DNA sequences incorporated in larger DNA molecules will have a maximal size range dependent on what can stably be incorporated in a respective DNA molecule. The skilled person will know and understand that different cloning and/or expression vectors can accommodate different sizes of DNA inserts. Table 1 provides an overview of different cloning/expression vector systems envisaged for use in certain embodiments of the present invention with their respective insert sizes according to these embodiments.If the composition comprises DNA of an artificial DNA construct (a vector, in particular a cloning vector), it is preferred that the artificial DNA construct is selected from a plasmid, a cosmid, a fosmid, or an artificial chromosome, such as a bacterial artificial chromosome, a yeast artificial chromosome, or a fungus artificial chromosome.

**Table 1**

| Free fragmented DNA | Plasmid incorporated | Fosmid incorporated | BAC incorporated | YAC incorporated | Incorporated in natural chromosomes |
|---|---|---|---|---|---|
| 50-3000 bp | 0.3-15 kb | 15-60 kb | 40-340 kb | 250 kb - 2Mb | 0.3 kb - 2 Mb |

The composition preferably comprises fragments of chromosomal DNA from the source species and/or the hosts species. The skilled person will know the extent of the term "chromosomal DNA" and will be able to provide chromosomal DNA of a certain organism from which DNA fragments may be derived.

The species from which the inhibitory DNA sequences are derived is referred to in this description and in the claims as the "source species". It will be clear that the term "source species" is used as a reference term. The term is used for easy distinction of the source species from the "host species" and the "target species" which will be discussed further below.

According to certain embodiments, in the composition of the invention, the DNA molecules may be free DNA. The term "free DNA" refers to the state wherein the DNA fragments are not incorporated in an envelope, such as a cell or virus particle. Free DNA fragments may be provided in any suitable form. For example, free DNA fragments may be in solution in a suitable solvent, such as water or an aqueous mixture. The solvent may be part of a single-phase solution or of a multiphase, such as an emulsion or a dispersion. Alternatively, the free DNA fragments may be present in the composition of the invention in a solid. The solid containing the free DNA may a solid in a single phase or a solid in a multiphase, such as a dispersion.

According to certain embodiments, inthe composition of the invention the mixture of DNA molecules comprises chimeric DNA molecules wherein source species DNA sequences are flanked on at least one end by non-source species DNA sequences. The skilled person will know the meaning of the term "chimeric DNA molecule" and will understand that this term refers to a DNA molecule comprising DNA sequences of at least two different sources, such as from a first species and from at least one different source, such as from a second species differing from the first species. The DNA sequence of the source species and the at least one flanking non-source species DNA sequence are thus in a single DNA molecule. A chimeric DNA molecule may alternatively be referred to as a hybrid DNA molecule.

In the chimeric DNA molecules the source species DNA sequences are flanked on at least one end by non-source species DNA sequences. The term "at least one" in the description of the present invention is equivalent to "a number of", and each time either of these terms is used it means "one or more" and vice versa. In certain preferred embodiments the terms mean a plurality, such as 2, 3, 4, 5, 6, 7, 8, 9 or 10, if appropriate. The skilled person will know that due to the structure of DNA molecules source species DNA sequence can be flanked in a chimeric DNA molecule by non-source species DNA sequences at most on two ends.

As the skilled person will understand, flanking DNA sequences occupy a position at each other's side. Thus the term "flanked" is used in the definition of the present invention as meaning to "border" or to "adjoin" or to "abut" and is interchangeable with these terms. Although this is already implied by the context wherein the term "flanked" (and any of its equivalent terms) is used, for clarity it is expressly stated that the source species DNA sequences are connected to the at least one flanking non-source species in a single DNA molecule.

Non-source species DNA sequences are DNA sequences having an origin other than the source species. Non-source species DNA sequences may be naturally occurring DNA sequences from a species different from the source species, such as chromosomal DNA sequences, such as from the host species. Alternatively, non-source species DNA sequences may be artificial DNA sequences, such as from an artificial DNA construct, such as a DNA sequence from an artificial plasmid, a cosmid, a fosmid or an artificial chromosome. Within the context of the present invention, an artificial chromosome may be selected from a yeast or fungus artificial chromosome or a bacterial artificial chromosome.

Chimeric DNA molecules comprising source species DNA sequences flanked on at least one end by non-source species DNA sequences can be obtained with procedures known to the skilled person. According to certain embodiments, where the source species DNA sequences are chromosomal DNA sequences and the non-sources species DNA sequences are sequences from an artificial plasmid, chromosomal DNA of the source species (possibly together with DNA of other parts of the total genome) may be isolated and (randomly) fragmented, for example by (partial) restriction enzyme digestion. The digested source species DNA may subsequently be ligated in the artificial plasmid cut with a compatible restriction enzyme, thus producing a population of chimeric plasmids having different partial sequences (fragments) of source species DNA incorporated. Compositions comprising such a mixed population of plasmids and comparable or derived compositions, for example wherein the plasmids are (randomly) fragmented into DNA fragments, are embodiments of compositions of the invention. According to other embodiments, where the source species DNA sequences are chromosomal DNA sequences and the non-sources species DNA sequences are also chromosomal DNA sequences, chromosomal DNA of the source species (possibly together with DNA of other parts of the total genome) may be isolated and (randomly) fragmented, for example by (partial) restriction enzyme digestion, sonication or other means of mechanical shearing, and the fragmented source species DNA may be contacted with cells of a host species competent of absorbing and incorporating foreign DNA fragments in their genome. Scientific knowledge about horizontal (or lateral) gene transfer between organisms of different species is growing and in particular for microorganisms, especially prokaryotes, it has been shown that they are capable of absorbing and incorporating foreign DNA in their genome, including in their chromosomal DNA. Furthermore, transposable elements (or transposons) may facilitate DNA transfer between cells of different species and source species DNA sequences could be incorporated in a transposable element to facilitate incorporation in the genome of a host species. Where in the process of horizontal gene transfer foreign DNA is incorporated in the DNA of a receiving organism, chimeric DNA molecules are formed, wherein the foreign (source) DNA is flanked by DNA of the receiving (host) organism. Compositions comprising such a mixed population of host organisms having incorporated in their chromosomal DNA source species DNA sequences and comparable or derived compositions, for example wherein the host species chromosomal DNA is isolated (possibly together with DNA of other parts of the total genome) and (randomly) fragmented into DNA fragments, are therefore alternative embodiments of compositions of the invention.

It should be understood that the chimeric region of the chimeric DNA molecules (the region where the source species DNA sequences adjoin with non-source species DNA sequences) may be very small in comparison to the length of the source species DNA sequences and/or the length of the non-source species DNA sequences. Thus, when chimeric DNA molecules are fragmented, there may be a high likely hood that DNA fragments contain only source species DNA sequences or non-source species DNA sequences. Thus the number of chimeric DNA fragments may be very small.

The source species preferably is selected such that fragmented DNA sequences derived therefrom are inhibitory for a target species. As is clear for the skilled person since the publication of WO2014/020624, DNA fragments that are inhibitory for a target species may be derived from the target species itself or from a phylogenetically similar species.

In the context of the present invention, the source species thus may be selected from a target species or from a species phylogenetically similar to a target species. As the skilled person will understand the term "inhibition" in the context of inhibition of a target species refers to interference with, slowing down or even stopping development of target species individuals and/or the population of the target species. It may be expected that the inhibiting effect of inhibitory self-DNA works via interfering with the physiology of the target species at the cellular level. Self-DNA should be understood to mean DNA of a species or of a phylogenetically similar species.

The target species may be a species selected from plants, fungi, insects, yeasts, bacteria, archaea, algae, nematodes, acari, viruses and prostists, preferably a species which may cause health and/or economic and/or environmental damage. Such a target species may for example be a disease associated species, such as a pathogenic species a parasitic, species or a species serving as a disease vector, or may be an infesting species, or may be a species associated with deterioration of products, such as of food products and/or of cosmetic products and/or of pharmaceutical products and/or of other products comprising organic matter. Disease associated species may cause and/or facilitate the spreading of a diseases to an animal, in particular to a human and/or a livestock animal, or to a plant, in particular to a crop. An infesting species may be any species, such as an insect species, or a higher animal species, or plant species, whereof individuals are present in a place or site (the target area) in larger than desired numbers. Infesting species at least cause nuisance and may (potentially) cause damage or harm. An infesting species according to certain embodiments may thus be considered a pest. As the skilled person will understand, biological species may cause deterioration of products in many ways. Often the mere presence of individuals of a species are undesired, such as in food products, in particular when the species can produce off-flavours and/or toxins. In addition, conversion of organic matter present in a product may lead to a reduced product quality, such as by the product not conforming to product specifications and/or by a (partial) loss of product function. It will be clear for the skilled person that the terms "disease associated species", "pathogenic species", "parasitic species", "species serving as a disease vector", "infesting species" and "species associated with deterioration of products" are not mutually excluding and that there is a degree of overlap between two or more of these terms. The terms are merely used to identify domains where inhibition of a target species may be beneficial and where the present invention preferably is employed.

When the target species is selected as a pathogenic species, it may be selected from Acinetobacter baumannii, or Actinomyces israelii, or Actinomyces gerencseriae, or Propionibacterium propionicus, or Trypanosoma brucei, or HIV (Human immunodeficiency virus), or Entamoeba histolytica, or Anaplasma species, or Angiostrongylus species, or Anisakis species, or Bacillus anthracis, or Arcanobacterium haemolyticum, or Junin virus, or Ascaris lumbricoides, or Aspergillus species, or species of the Astroviridae family, or Babesia species, or Bacillus cereus, or Bacteroides species, or Balantidium coli, or Bartonella, or Baylisascaris species, or BK virus, or Piedraia hortae, or Blastocystis species, or Blastomyces dermatitidis, or Machupo virus, or Clostridium botulinum, or Sabiá virus, or Brucella species, or Yersinia Pestis, or Burkholderia cepacia, or other Burkholderia species, or Mycobacterium ulcerans, or Caliciviridae family, or Campylobacter species, or Candida albicans, or other Candida species, or Capillaria philippinensis, or Capillaria aerophila, or Bartonella bacilliformis, or Bartonella henselae, or Group A Streptococcus spp., or Staphylococcus spp., or Trypanosoma cruzi, or Haemophilus ducreyi, or Varicella zoster virus (VZV), or Alphavirus, or Chlamydia trachomatis, or Chlamydophila pneumoniae, or Vibrio cholerae, or Fonsecaea pedrosoi, or Batrachochytrium dendrabatidis, or Clonorchis sinensis, or Clostridium difficile, or Coccidioides immitis and Coccidioides posadasii, or Colorado tick fever virus (CTFV), or rhinovirus spp., or coronaviruses, or PRNP, or Crimean-Congo hemorrhagic fever virus, or Cryptococcus neoformans, or Cryptosporidium species, or Ancylostoma braziliense,, or Cyclospora cayetanensis, or Taenia solium, or Cytomegalovirus spp.,, or Dengue viruses (DEN-1, DEN-2, DEN-3 and DEN-4),, or Flavivirus spp.,, or green algae,, or Desmodesmus armatus, or Dientamoeba fragilis, or Corynebacterium diphtheriae, or Diphyllobothrium, or Dracunculus medinensis, or Ebolavirus (EBOV), or Echinococcus species, or Ehrlichia species, or Enterobius vermicularis, or Enterococcus species, or Enterovirus species, or Rickettsia prowazekii, or Parvovirus B 19, or Human herpesvirus 6 (HHV-6),, or Human herpesvirus 7 (HHV-7), or Fasciola hepatica, or Fasciola gigantica, or Fasciolopsis buski, or PRNP, or Filarioidea superfamily, or Clostridium perfringens, or multiple, or Fusobacterium species, or Clostridium perfringens, or other Clostridium species, or Geotrichum candidum, or Giardia lamblia, or Burkholderia mallei, or Gnathostoma spinigerum,, or Gnathostoma hispidum, or Neisseria gonorrhoeae, or Klebsiella granulomatis, or Streptococcus pyogenes, or Streptococcus agalactiae, or Haemophilus influenzae, or Enteroviruses, such as Coxsackie A virus and Enterovirus 71 (EV71),, or Sin Nombre virus, or Heartland virus, or Helicobacter pylori, or Escherichia coli O157:H7, O111 and O104:H4,, or species from the Bunyaviridae family, or Hendra virus, or Hepatitis A virus, or Hepatitis B virus, or Hepatitis C virus, or Hepatitis D Virus, or Hepatitis E virus, or Herpes simplex virus 1 and 2 (HSV-1 and HSV-2), or Histoplasma capsulatum, or Ancylostoma duodenale and Necator americanus, or Human bocavirus (HBoV), or Ehrlichia ewingii, or Anaplasma phagocytophilum, or Human metapneumovirus (hMPV), or Ehrlichia chaffeensis, or One of the Human papillomaviruses, or Human parainfluenza viruses (HPIV), or Hymenolepis nana and Hymenolepis diminuta, or Epstein-Barr virus (EBV), or Orthomyxoviridae family, or Isospora belli, or Kingella kingae, or Lassa virus, or Legionella pneumophila, or Legionella pneumophila, or Leishmania species, or Mycobacterium leprae, or Mycobacterium lepromatosis, or Leptospira species, or Listeria monocytogenes, or Borrelia burgdorferi, or Borrelia garinii,, or Borrelia afzelii, or Wuchereria bancrofti,, or Brugia malayi, or Lymphocytic choriomeningitis virus (LCMV), or Plasmodium species, or Marburg virus, or Measles virus, or Middle East respiratory syndrome coronavirus, or Burkholderia pseudomallei, or Neisseria meningitidis, or Metagonimus yokagawai, or Microsporidia phylum, or Molluscum contagiosum virus (MCV), or Monkeypox virus, or Mumps virus, or Rickettsia typhi, or Mycoplasma pneumoniae, or Mycoplasma genitalium, or Actinomycetoma spp., or Eumycetoma spp., or Chlamydia trachomatis and Neisseria gonorrhoeae, or Nipah virus, or Norovirus, or Nocardia asteroids, or other Nocardia species, or Onchocerca volvulus, or Opisthorchis viverrini, or Opisthorchis felineus, or Paracoccidioides brasiliensis, or Paragonimus westermani, or other Paragonimus species, or Pasteurella species, or Pediculus humanus capitis, or Pediculus humanus corporis, or Pthirus pubis, or Bordetella pertussis, or Yersinia pestis, or Streptococcus pneumoniae, or Pneumocystis jirovecii, or Poliovirus, or Prevotella species, or Naegleria fowleri, or JC virus, or Chlamydophila psittaci, or Coxiella burnetii, or Rabies virus, or Borrelia hermsii, Borrelia recurrentis, oe other Borrelia species, or Respiratory syncytial virus (RSV), or Rhinosporidium seeberi, or Rhinovirus, or Rickettsia species, or Rickettsia akari, or Rift Valley fever virus, or Rickettsia rickettsii, or Rotavirus, or Rubella virus, or Salmonella species, or SARS coronavirus, or Sarcoptes scabiei, or Group A Streptococcus species, or Schistosoma species, or Shigella species, or Varicella zoster virus (VZV), or Variola major, or Variola minor, or Sporothrix schenckii, or Staphylococcus species,, or Strongyloides stercoralis, or Measles virus, or Treponema pallidum, or Taenia species, or Clostridium tetani, or Trichophyton species, or Trichophyton tonsurans, or Epidermophyton floccosum, or Trichophyton rubrum, or Trichophyton mentagrophytes, or Trichophyton rubrum, or Hortaea werneckii, or Malassezia species, or Toxocara canis, or Toxocara cati, or Toxoplasma gondii, or Chlamydia trachomatis, or Trichinella spiralis, or Trichomonas vaginalis, or Trichuris trichiura, or Mycobacterium tuberculosis, or Francisella tularensis, or Salmonella enterica, or serovar typhi, or Rickettsia, or Ureaplasma urealyticum, or Coccidioides immitis, or Coccidioides posadasii, or Venezuelan equine encephalitis virus, or Guanarito virus, or Vibrio vulnificus, or Vibrio parahaemolyticus, or West Nile virus, or Trichosporon beigelii, or Yersinia pseudotuberculosis, or Yersinia enterocolitica, or Yellow fever virus, or Zeaspora fungus, or Zika virus, or Mucorales, or Entomophthorales.

It should be understood that inhibition of pathogenic target species need not be in or on an animal (including a human) body. Instead the inhibition may also be outside the context of an animal body. For example for inhibiting the target species in a (*in vitro*) culture. Selection of pathogenic target species from topical pathogenic and/or topical target species is preferred, in particular topical pathogenic target species from the list presented directly above. The skilled person will understand that the term topical in the context of human and veterinary medicine means pertaining to a particular surface of the body, in particular the skin or mucous membranes (mucosa). Topical pathogenic target species should thus be considered to be associated with the skin and/or nails and/or with mucous membranes, including the mucous membranes of the eye, the mouth, the vagina, the urinary tract, the gastrointestinal tract, the airways, including the lungs. The term topical thus is not limited to the exterior surface of an animal body, but includes reference to internal surfaces, such as the lungs and gastrointestinal tract. Topical pathogenic target species most preferably are skin pathogens and/or nail pathogens and/or are mucosal pathogens. Within the context of the present invention selection of pathogenic target species from archaea, bacteria, fungi (including yeasts) and protists is further preferred, in particular archaea, bacteria, fungi (including yeasts) and protists from the list presented directly above.

When the target species is selected as a parasitic species, it may be selected from Acanthamoeba spp. or Balamuthia mandrillaris or Babesia B. divergens or B. bigemina or B. equi or B. microfti or B. duncani or Balantidium coli or Blastocystis spp. or Cryptosporidium spp. or Cyclospora cayetanensis or Dientamoeba fragilis or Entamoeba histolytica or Giardia lamblia or Isospora belli or Leishmania spp. or Naegleria fowleri or Plasmodium falciparum or Plasmodium vivax or Plasmodium ovale curtisi or Plasmodium ovale Wallikeri or Plasmodium malariae or Plasmodium knowlesi or Rhinosporidium seeberi or Sarcocystis or bovihominis,Sarcocystis or suihominis or Toxoplasma gondii or Trichomonas vaginalis or Trypanosoma brucei or Trypanosoma cruzi or Cestoda or Taenia or multiceps or Diphyllobothrium latum or Echinococcus or granulosus or Echinococcus or multilocularis or E. vogeli or E. oligarthrus or Hymenolepis nana or Hymenolepis diminuta or Taenia saginata or Taenia solium or Bertiella mucronata or Bertiella studeri or Spirometra or Erinaceieuropaei or Schistosoma haematobium or Schistosoma japonicum or Schistosoma mekongi or Echinostoma echinatum or Trichobilharzia regenti or Schistosomatidae or Ancylostoma or duodenale or Necator or americanus or Angiostrongylus or costaricensis or Anisakis or Ascaris sp. Ascaris or lumbricoides or Baylisascaris or procyonis or Brugia malayi or Brugia or timori or Dioctophyme renale or Dracunculus or medinensis or Enterobius or vermicularis or Enterobius gregorii or Gnathostoma or spinigerum or Gnathostoma or hispidum or Halicephalobus or gingivalis or Loa loa filaria or Mansonella or Streptocerca or Onchocerca volvulus or Strongyloides or stercoralis or Thelazia or californiensis or Thelazia callipaeda or Toxocara canis or Toxocara cati or Trichinella spirali s.

Similar to what is noted in connection to pathogenic target species, it should be understood that inhibition of parasitic target species need not be in or on an animal (including a human) body. Instead the inhibition may also be outside the context of an animal body. For example for inhibiting the target species in a culture. Selection of parasitic target species from skin parasites and/or gastrointestinal parasites and/or mucosal parasites is preferred, in particular selected from the list presented directly above. Selection of parasitic target species from protists or nematodes is preferred, in particular protists and nematodes from the list presented directly above.

According to certain embodiments, the target species may be selected from a species pathogenic for a plant, such as a plant pathogen selected from fungi or Oomycetes or bacteria or viruses or protists or Fusarium spp. or Thielaviopsis spp. or Verticillium spp. or Magnaporthe spp. or Magnaporthe grisea or Sclerotinia spp. or Sclerotinia sclerotiorum or Phytophtora spp. or Pythium spp. Plasmodiophora spp. or Spongospora spp. or phytopathogenic bacilli or Erwinia spp. or Agrobacterium spp. or Burkholderia spp. or Proteobacteria or Xanthomonas spp. or Pseudomonas spp. or Phytoplasma spp. or Spiroplasma spp..

When the target species is selected as an infesting species, it may be selected from an agricultural pest, such as an agricultural pest arthropod such as a species selected from Acalymma or Acyrthosiphon kondoi or Acyrthosiphon gossypii or Acyrthosiphon pisum or African armyworm or Africanized bee or Agromyzidae or Agrotis ipsilon or Agrotis munda or Agrotis orthogonia or Agrotis porphyricollis or Akkaia taiwana or Aleurocanthus woglumi or Aleyrodes proletella or Alphitobius diaperinus or Alsophila aescularia or Altica chalybea or Anasa tristis or Anisoplia austriaca or Anthonomus pomorum or Anthonomus signatus or Aonidiella aurantii or Aonidiella citrina or Aonidiella orientalis or Apamea apamiformis or Apamea niveivenosa or Aphid or Aphis gossypii or Aphis nasturtii or Apple maggot or Argentine ant or Army cutworm or Fall armyworm or Arotrophora arcuatalis or Ash whitefly or Astegopteryx bambusae or Astegopteryx insularis or Astegopteryx minuta or Asterolecanium or Asterolecanium coffeae or Atherigona reversura or Athous haemorrhoidalis or Aulacophora or Aulacorthum solani or Australian plague locust or Bactericera cockerelli or Bactrocera or Bactrocera correcta or Bagrada hilaris or Knulliana or Beet armyworm or Black bean aphid or Blepharidopterus chlorionis or Bogong moth or Boll weevil or Bollworm or Brevicoryne brassicae or Brown locust or Brown marmorated stink bug or Brown planthopper or Cabbage moth or Cabbage worm or Callosobruchus maculatus or Carrot fly or Cerataphis brasiliensis or Ceratitis aliena or Ceratitis andranotobaka or Ceratitis capitata or Ceratitis flexuosa or Ceratitis grahami or Ceratitis ovalis or Ceratitis penicillata or Ceratitis rosa or Ceratoglyphina bambusae or Ceratopemphigus zehntneri or Ceratovacuna lanigera or Cereal leaf beetle or Chaetosiphon tetrarhodum or Chlorops pumilionis or Citrus long-horned beetle or Coccus hesperidum or Coccus viridis or Codling moth or Coffee borer beetle or Colias eurytheme or Colorado potato beetle or Common blossom thrips or Confused flour beetle or Cotton bollworm or Crambus or Cucumber beetle or Curculio elephas or Curculio nucum or Curculio occidentis or Cutworm or Cyclocephala borealis or Dargida diffusa or Dasineura brassicae or Date stone beetle or Delia (fly) or Delia antiqua or Delia floralis or Delia platura or Delia radicum or Dermestes ater or Dermolepida albohirtum or Desert locust or Diabrotica or Diabrotica balteata or Diabrotica speciosa or Diamondback moth or Diaphania indica or Diaphania nitidalis or Diaphorina citri or Diaprepes abbreviatus or Diatraea saccharalis or Differential grasshopper or Diparopsis castanea or Dociostaurus maroccanus or Drosophila suzukii or Dryocosmus kuriphilus or Dysaphis crataegi or Dysmicoccus brevipes or Earias perhuegeli or Epicauta vittata or Epilachna or Epitrix cucumeris or Epitrix tuberis or Erionota thrax or Eriosoma lanigerum or Eriosomatinae or Euleia heraclei or Eumetopina flavipes or European corn borer or Eurydema oleracea or Eurygaster integriceps or Ferrisia virgata or Forest bug or Frankliniella tritici or Galleria mellonella or Garden dart or Geoica lucifuga or Glassy-winged sharpshooter or Greenhouse whitefly or Greenidea artocarpi or Greenidea formosana or Greenideoida ceyloniae or Gryllotalpa orientalis or Gryllotalpa vinae or Gypsy moths in the United States or Helicoverpa armigera or Helicoverpa gelotopoeon or Helicoverpa punctigera or Helicoverpa zea or Heliothis virescens or Henosepilachna or Henosepilachna vigintioctomaculata or Henosepilachna vigintioctopunctata or Hessian fly or Heteronychus arator or Hyalopterus pruni or Hysteroneura setariae or Icerya purchasi or Ipuka dispersum or Jacobiasca formosana or Kaltenbachiella elsholtriae or Kaltenbachiella japonica or Khapra beetle or Lampides boeticus or Leaf miner or Lema daturaphila or Lepidiota consobrina or Lepidosaphes beckii or Lepidosaphes ulmi or Leptocybe invasa or Leptoglossus zonatus or Leptopterna dolabrata or Lesser wax moth or Leucoptera (moth) or Leucoptera caffeina or Light brown apple moth or Light brown apple moth controversy or Lipaphis erysimi or Liriomyza huidobrensis or Lissorhoptrus oryzophilus or Listronotus bonariensis or Long-tailed skipper or Lygus or Lygus hesperus or Macrodactylus subspinosus or Macrosiphoniella pseudoartemisiae or Macrosiphoniella sanborni or Macrosiphum euphorbiae or Maize weevil or Manduca sexta or Matsumuraj a capitophoroides or Mayetiola hordei or Mealybug or Megacopta cribraria or Melanaphis sacchari or Melittobia australica or Metcalfa pruinosa or Mexican bean beetle or Micromyzus judenkoi or Micromyzus kalimpongensis or Micromyzus niger or Moth or Leek moth or Mythimna unipuncta or Myzus ascalonicus or Myzus boehmeriae or Myzus cerasi or Myzus obtusirostris or Myzus ornatus or Myzus persicae or Nematus or Nematus leucotrochus or Nematus ribesii or Nematus spiraeae or Neomyzus circumflexus or Neotoxoptera oliveri or Nezara viridula or Oak processionary or Oebalus pugnax or Olive fruit fly or Ophiomyia simplex or Opisina arenosella or Opomyza or Opomyza florum or Opomyzidae or Orseolia oryzae or Oryzaephilus mercator or Oscinella frit or Ostrinia furnacalis or Oxycarenus hyalinipennis or Papilio demodocus or Paracoccus marginatus or Paratachardina pseudolobata or Paropsisterna selmani or Patanga succincta or Pemphigus betae or Pentalonia nigronervosa or Pentatomoidea or Peridroma saucia or Phorodon humuli or Phthorimaea operculella or Phyllophaga or Phyllotreta nemorum or Phylloxeridae or Phylloxeroidea or Phytomyza horticola or Pieris brassicae or Pink bollworm or Planococcus citri or Platynota idaeusalis or Plum curculio or Prionus californicus or Pristiphora or Pseudoregma bambucicola or Pseudotheraptus wayi or Psylliodes chrysocephala or Ptinus fur or Pyralis farinalis or Raphidopalpa foveicollis or Red imported fire ant or Red locust or Rhagoletis cerasi or Rhagoletis indifferens or Rhagoletis mendax or Rhodobium porosum or Rhopalosiphoninus latysiphon or Rhopalosiphum maidis or Rhopalosiphum padi or Rhopalosiphum rufiabdominale or Rhyacionia frustrana or Rhynchophorus ferrugineus or Rhynchophorus palmarum or Rhynchophorus vulneratus or Rhyzopertha or Rice moth or Russian wheat aphid or Saissetia oleae or San Jose scale or Scale insect or Schistocerca americana or Schizaphis graminum or Schizaphis hypersiphonata or Schizaphis minuta or Schizaphis rotundiventris or Schoutedenia lutea or Sciaridae or Scirtothrips dorsalis or Scutelleridae or Scutiphora pedicellata or Serpentine leaf miner or Setaceous Hebrew character or Shivaphis celti or Silver or Silverleaf whitefly or Sinomegoura citricola or Sipha flava or Sitobion avenae or Sitobion lambersi or Sitobion leelamaniae or Sitobion miscanthi or Sitobion pauliani or Sitobion phyllanthi or Sitobion wikstroemiae or Sitona lepidus or Sitona lineatus or Small hive beetle or Southwestern corn borer or Soybean aphid or Spodoptera cilium or Spodoptera litura or Spotted cucumber beetle or Spotted lanternfly or Squash vine borer or Stemborer or Stenotus binotatus or Strauzia longipennis or Striped flea beetle or Sunn pest or Sweetpotato bug or Synanthedon exitiosa or Tarnished plant bug or Tecia solanivora or Tetranychus urticae or other Tretranychus spp., Tetraneura nigriabdominalis or Tetraneura yezoensis or Thrips or Thrips angusticeps or Thrips palmi or Thrips simplex or Thrips tabaci or Thysanoplusia orichalcea or Tinocallis kahawaluokalani or Toxoptera aurantii or Toxoptera citricida or Toxoptera odinae or Trichobaris trinotata or Trioza erytreae or Turnip moth or Tuta absoluta or Uroleucon minutum or Varied carpet beetle or Vesiculaphis caricis or Virachola isocrates or Waxworm or Western corn rootworm or Western flower thrips or Wheat fly or Wheat weevil or Whitefly or Winter moth or Xylotrechus quadripes or Zygogramma exclamationis. According to certain embodiments, selection of a target species from the order Lepidoptera is preferred, in particular selected from the family Tortricidae, such as from the genus Choristoneura, in particular Choristoneura orae, Choristoneura fumiferana or Choristoneura freemani, or selected from the family Noctuidae, such as the genus Spodoptera, in particular Spodoptera frugiperda, Spodoptera litura, Spodoptera litoralis, Spodoptera cilium or Spodoptera ornithogalli, or selected from the family Pyralidae, such as from the genus Plodia or Ephestia, or selected from other species from this order motioned in the list directly above. An agricultural pest species may also be selected from a phytophagous terrestrial gastropod species.

A pest species may further be selected from a disease vector, such as a disease vector selected from arthropods. The disease vector may be involved in the spreading of an animal disease, including a human disease, or may vector a plant disease. Diseases vectors vectoring animal diseases may be selected from blood feeding (haematophagous) or haemolymph feeding arthropods, preferably a blood feeding arthropod, for example selected from the family Culicidae, such as from the genus *Aedes,* or the family Ceratopogonidae, such as form the genus *Culicoides,* or the family Tabanidae, or from the family Simuliidae, such as from the genus *Austrosimulium,* or the family Glossinidae, such as from the genus Glossina, or the family Triatominae, such as *Triatoma infestans* or *Rhodnius prolixus,* or from the Siphonoptera, such as from the Publicidae, or from the Phthiraptera, such as from the genus *Pediculus,* or from the family Ixodidae, or from the family Argasidae.

Arthropod vectors involved in spreading plant diseases may be selected from Acyrthosiphon pisum or Agromyzidae or Anastrepha grandis or Anastrepha obliqua or Anthomyiidae or Aphids or Bark beetles or Beet leafhoppers or Brevicoryne brassicae or Cacopsylla melanoneura or Cacopsylla ulmi or Ceratitis podocarpi or Chaetosiphon fragaefolii or Cicadulina or Cicadulina mbila or Common brown leafhopper or Cryptococcus fagisuga or Curculionidae or Diabrotica balteata or Empoasca decedens or Eumetopina flavipes or Euscelis plebejus or Frankliniella tritici or Glassy-winged sharpshooter or Haplaxius crudus or Hyalesthes obsoletus or Hylastes ater or Leaf beetle or Leafhopper or Lipaphis erysimi or Macrosteles quadrilineatus or Mealybug or Melon fly or Molytinae or Pegomya hyoscyami or Pissodes or Pissodes strobi or Pissodini or Planthopper or Pseudococcus maritimus or Pseudococcus viburni or Psylla pyri or Psyllidae or Rabdophaga clavifex or Rhynchophorus palmarum or Scaphoideus titanus or Scirtothrips dorsalis or Silverleaf whitefly or Tephritidae or Thripidae or Thrips palmi or Tomicus piniperda or Toxoptera citricida or Treehopper or Triozidae or Western flower thrips or Xyleborus glabratus.

According to certain preferred embodiments a pest species is selected as a nematode species parasitic to plants, in particular selected from the genus Meloidogyne, such as M. arenaria, M. incognita, M. javanica, or M. hapla, or selected from the genus Hetrodera, such as Heterodera glycines, or Heterodera avenae and H. filipjevi, or selected from the genus Globodera, such as Globodera pallida, or G. rostochiensis, or selected from the genus Pratylenchus, such as P. penetrans, P. thornei, P. neglectus, P. zeae, P. vulnus or P. coffeae, or selected from the genus Radopholus, such as Radopholus similis.

According to certain embodiments, infesting species may be selected from weed species. Weed species considered as target species within the present invention are for example weed species from the Alismataceae or Apiaceae or Asteraceae or Amaranthaceae or Cactaceae or Caryophyllaceae or Chenopodiaceae or Caulerpaceae or Commelinaceae or Poaceae or Portulacaceae or Euphorbiaceae or Fabaceae (Leguminosae) or Rubiaceae or Hydrocharitaceae or Azollaceae or Salviniaceae or Iridaceae or Liliaceae or Pontederiaceae or Melastomataceae or Myrtaceae or Polygonaceae or Lygodiaceae or Rosaceae or Acanthaceae or Orobanchaceae or Scrophulariaceae or Convolvulaceae or Cuscutaceae or Solanaceae or Sparganiaceae.

Specific weed species considered as target species may be selected from Sagittaria sagittifolia Linnaeus or Heracleum mantegazzianum Sommier & Levier or Ageratina adenophora (Spreng.) King & H.E. Robins. or Ageratina riparia (Regel) King & H.E. Robins. or Arctotheca calendula (L.) Levyns or Carthamus oxyacanthus M. Bieberstein or Crupina vulgaris Cass. or Inula britannica L. or Mikania cordata (Burm. f.) B.L. Robins. or Mikania micrantha Kunth or Onopordum acaulon L. or Onopordum illyricum L. or Senecio inaequidens DC. or Senecio madagascariensis Poir. or Tridax procumbens L. or Alternanthera sessilis (L.) R. Br. ex DC. or Opuntia aurantiaca Lindley or Drymaria arenarioides Humboldt & Bonpland or Salsola vermiculata L. or Caulerpa taxifolia (Vahl) C. Agardth or Commelina benghalensis L. or Avena sterilis Linnaeus or Chrysopogon aciculatus (Retz.) Trin. or Digitaria abyssinica (A. Rich) Stapf or Digitaria velutina (Forsk.) Beauv. or Imperata brasiliensis Trinius or Imperata cylindrica (L.) Beauv. or Ischaemum rugosum Salisbury or Leptochloa chinensis (L.) Nees or Nassella trichotoma Hackel ex Arech. or Oryza longistaminata A. Chev. & Roehr. or Oryza punctata Kotzchy ex Steud. or Oryza rufipogon Griffiths or Paspalum scrobiculatum Linnaeus or Pennisetum clandestinum Hochst. ex Chiov. or Pennisetum macrourum Trinius or Pennisetum pedicellatum Trinius or Pennisetum polystachion (Linnaeus) Schultes or Rottboellia cochinchinensis (Lour.) W.D. Clayton or Saccharum spontaneum L. or Setaria pumila ssp. pallidefusca (Schumacher) B.K. Simon or Urochloa panicoides Beauvois or Euphorbia terracina L. or Acacia nilotica (L.) Willd. ex Delile or Galega officinalis L. or Mimosa diplotricha C. Wright ex Sauvalle or Mimosa pigra L. or Prosopis alpataco R. A. Philippi or Prosopis argentina Burkart or Prosopis articulata S. Watson or Prosopis burkartii Muñoz or Prosopis caldenia Burkart or Prosopis calingastana Burkart or Prosopis campestris Griesbach or Prosopis castellanosii Burkart or Prosopis denudans Bentham or Prosopis elata (Burkart) Burkart or Prosopis farcta (Banks & Soland.) J.F. Macbr. or Prosopis ferox Griesbach or Prosopis fiebrigii Harms or Prosopis hassleri Harms ex Hassler or Prosopis humilis Gillies ex Hooker & Arnott or Prosopis kuntzei Harms ex Hassler or Prosopis pallida (Humb. & Bonpl. ex Willd.) Kunth or Prosopis palmeri S. Watson or Prosopis reptans Benth. or Prosopis rojasiana Burkart or Prosopis ruizlealii Burkart or Prosopis ruscifolia Griesbach or Prosopis sericantha Gillies ex Hook. & Arn. or Prosopis strombulifera (Lamarck) Bentham or Prosopis torquata (Cavan. ex Lagasca y Segura) DC. or Spermacoce alata Aubl. or Hydrilla verticillata (L. f.) Royle or Lagarosiphon major (Ridley) Moss or Ottelia alismoides (Linnaeus) Pers. or Azolla pinnata R. Brown or Salvinia auriculata Aublet or Salvinia biloba Raddi or Salvinia herzogii de la Sota or Salvinia molesta D. S. Mitchell or Moraea collina Thunb. or Moraea flaccida (Sweet) Steud. or Moraea miniata Andrews or Moraea ochroleuca (Salisb.) Drapiez or Moraea pallida (Baker) Goldblatt or Asphodelus fistulosus Linnaeus or Eichhornia azurea (Swartz) Kunth or Monochoria hastata (L.) Solms or Monochoria vaginalis (Burm. f) K. Presl ex Kunth or Melastoma malabathricum L. or Melaleuca quinquenervia (Cav.) Blake or Emex australis Steinhall or Emex spinosa (Linnaeus) Campdera or Lygodium flexuosum (L.) Sw. (1801) (Mobot) or Lygodium microphyllum (Cav.) R. Br. or Rubus fruticosus L. or Rubus moluccanus L. or Hygrophila polysperma (Roxb.) T. Anders. or Aeginetia spp. L. or Alectra spp. Thunb. or Orobanche spp. (nonnative) L. or Limnophila sessiliflora (Vahl) Blume or Striga spp. Lour. or Ipomoea aquatica Forssk. or Cuscuta spp. L. or Lycium ferocissimum Miers or Solanum tampicense Dunal or Solanum torvum Sw. or Solanum viarum Dunal or Sparganium erectum L.

Species that cause product deterioration that may be selected as target species may be selected from spoilage microorganisms, such as selected from bacteria, such as Gram-negative rods, e.g. Pseudomonas spp., Shewanella spp., Gram-positive spore-formers, e.g. Bacillus spp., Clostridium spp., lactic acid bacteria and other Gram-positive bacteria, e.g. Brochothrix spp, Micrococcus spp., or Enterobacteriaceae, fungi, such as Zygomycetes, from the genus Penicillium, or the genus Aspergillus or yeasts such as Zygosaccharomyces spp, Saccharomyces spp., Candida spp., Dekkera (Brettanomyces) spp.. Alternatively, target species that cause product deterioration may be selected from stored product mites, such as selected from the Astigmata, such as selected from the Glycyphagidae, or the Carpoglyphidae.

According to certain preferred embodiments of the composition of the invention, the composition comprises cells from a number of species, the host species, differing from the source species, wherein DNA molecules are in host species cells.

As is already mentioned above, the term "host species" is merely used as a reference term. A host species in the context of the present invention in general is a species differing from the source species, preferably a phylogenetically dissimilar species, having incorporated intracellularly source species DNA sequences. Phylogenetically dissimilar (distant) species according to certain embodiments are species from different taxonomic orders, such as from different classes, different phylla, different kingdoms, or different domains. According to certain embodiments phylogenetically dissimilar species are species from different families, such from different orders, different classes, different phylla, different kingdoms, or different domains. Host species may be selected from any species capable of taking up and replicating foreign DNA of the source species, in particular fragments of source species DNA, such as fragments of source species incorporated in cloning and/or expression vectors. As is clear from this description, it is not required that an individual of the host species takes up and replicates the total of the genome of the source species. Instead the inhibitory effect of the self-DNA on the target species may be effected by a plurality of hosts species cells and/or individuals wherein respectively the different cells and/or individuals replicate a different part of the genome of the source species, as in a DNA library. A single cell of the population of host species cells thus contains only a fraction of the total of source species DNA sequences present in the composition. Host species may for example be selected from multicellular organisms or from microorganisms. For example cells of multicellular plants may be used as host species cells. As the skilled person will understand, source species DNA may be introduced into cells of multicellular plants, by various techniques, including particle bombardment (biolistics) of for example embryonic cell cultures, agrobacterium mediated transformation, transformation mediated by viral vector(s). When selected as a plant, the host species, may be selected from a crop, preferably a food crop, such as selected from cereals, corn, sugar beet, rapeseed, pea, soybean, an oil crop, such as rapeseed, Ethiopian mustard, sunflower, a starch crop, such as (sweet) sorghum, a fibre crop, such as flax or hemp, a lignocellulosic crop, such as reed canary grass, giant reed, switchgrass, miscanthus, cardoon, a short rotation forestry crop, such as willow, poplar, or eucalyptus.

Alternatively, helminths used in helminthic therapy such as *Trichuris suis, Necator americanus, Trichuris trichiura, Hymenolepis diminuta, Ascaris lumbricoides, Strongyloides stercoralis, Enterobius vermicularis* or *Hymenolepis nana* may be used as hosts species. A helminth species used as a host species preferably is a mutualistic species for a human, in particular *Trichuris suis, Necator americanus, Trichuris trichiura* or *Hymenolepis diminuta.* Events of horizontal gene transfer have been observed for nematode species (see amongst others PLoS Negl Trop Dis. 2007 Oct; 1(1): e35; doi: 10.1371/journal.pntd.0000035) and horizontal gene transfer could be induced for therapeutic helminths to incorporate source species DNA in their genome.

According to other alternative embodiments, the host species is selected from a microorganism. As the skilled person will know, a microorganisms is a microscopic organism, which may be a single-celled form or may exist in a colony of cells. Within the context of the present invention microorganisms are preferably selected from prokaryotes, such as from Archaea or bacteria, or from eukaryotes, such as from fungi (including yeasts) or from microphytes (microalgae). Host species selected from microorganisms may be selected from microorganisms that may be grown in a liquid culture, such as *E. coli,* in particular the strain DH5α or a derived strain, or the strain DH10B or a derived strain, or from a Saccharomyces species, such as *Saccharomyces cerevisiae,* such as the strain AB 1380 or a derived strain, or from Aspergillus species, such as Aspergillus niger, Aspergillus oryzae, or Aspergillus nidulans, or from a Bacillus species, or from a pseudomonas species, or from lactic acid bacteria, such as Lactobacillus spp., Leuconostoc spp., Pediococcus spp., Lactococcus spp., Enterococcus spp., or Streptococcus spp or from cyanobacteria, such as an *Arthrospira* species, in particular *Arthrospira plantenis* or *Arthrospira maxima.* According to certain embodiments, the microorganism may be an organism having a Generally Regarded as Safe (GRAS) status, as declared by the FDA . Alternatively soil microorganisms, preferably a mixed culture of different soil bacteria and/or soil fungi may be used as host species. Soil microorganism used as host species may be selected from Alphaproteobacteria spp., Betaproteobacteria spp., Deltaproteobacteria spp., Actinobacteria spp., Thermoleophilia spp., Rubrobacteria spp., Chloracidobacteria spp., Acidobacteria spp., or Solibacteres spp. According to certain preferred embodiments the host species is selected from a biocontrol agent, for example an entomopathogenic biocontrol agent, such as a microbial biocontrol agent, in particular a bacterial biocontrol agent such as *Bacillus thuringiensis* or *Bacillus subtillus,* or a fungal biocontrol agent, such as *Beauveria bassiana*, *Isaria fumosorosea*, *Lecanicillium* spp., or *Metarhizium* spp.. biocontrol agents may alternatively be selected from for example *Trichoderma* spp. or *Ampelomyces quisqualis.* The host species may further be selected from microbial plant growth stimulants, such as *Bacillus* species, *Trichoderma* species or Mycorrhyza species. According to certain highly preferred embodiments, when the host species is a microbial entomopathogenic biocontrol agent, the target species is an arthropod, prefererably an arthropod selected from an agricultural pest arthropod or Arthropod vectors involved in spreading plant diseases. Specific examples of organisms from these groups have been presented above.

As explained, in embodiments wherein the source species DNA sequences are in host species cells, the source species DNA sequences are either incorporated in artificial replicable DNA constructs, such as artificial plasmids or artificial chromosomes, introduced in the hosts species cells, or are incorporated in the natural genome of the hosts species cells. By this incorporation of the source species DNA sequences in artificial DNA constructs introduced into the host cells or in the natural genome of the hosts cells, the source species DNA sequences can be replicated by the DNA replication machinery of the host species cells together with the replication of host species DNA. The surprising discovery by the inventors of the present invention, that inhibitory DNA fragments of a species remain inhibitory when presented in association with DNA of a (production) host species, has opened new possibilities for producing and presenting self-inhibitory DNA fragments.

Incorporation of DNA sequences in DNA constructs and the introduction of DNA constructs into host cells has become standard practice in various fields of biological sciences. Thus the skilled person can readily apply such procedures within the present invention. In addition, it has become known that various organisms, can take-up DNA molecules from their environment and can incorporate these DNA molecules in their natural genome. In particular microorganisms, especially prokaryotes, are naturally capable of such horizontal or lateral gene transfer. In addition, certain known genetic engineering techniques, such as biolistics, depend on incorporation of DNA fragments in the natural genome of cells, such as plant cells.

Furthermore, transposable elements (or transposons) may facilitate DNA transfer between cells of different species.

It is preferred that the coverage of the genome of the source species in the mixture of DNA molecules is between 20-100%, such as 20-90%, 30-100%, 30-90%, 40-100%, 40-90%, 50-100%, 50-90%, preferably 60-100%, such as 60-90%, 70-100%, 70-90%, 80-100%, 80-90%. Based on experimental observations by the inventors, it can be derived that within these ranges there is good inhibitory activity of the source species DNA sequences in the composition of the invention. The skilled person will know and understand that the coverage of the source species genome in the mixture of DNA molecules is correlated to the percentage of source species DNA sequences, in particular chromosomal DNA sequences, that is present in the whole (population) of the DNA molecules present in the composition. In view of the fact that experimental results indicate that a fraction of the total genome is inhibitory, it may also be expected that (fragmented) cDNA sequences, which also represents a fraction of the total of DNA sequences of a genome, will have inhibitory activity on the species from which they are derived and on phylogenetically similar species. It is however preferred that the source species DNA sequences comprise non-coding sequences, if the source species genome comprises a substantial amount of such non-coding sequences, as based on the knowledge available in connection to chromosomal organisation, it may be expected that there is a higher species specificity in non-coding sequences. In view of thius it is preferred that the source species DNA sequences comprise randomly fragmented chromosomal DNA.

By replicating the source species DNA sequences in (chimeric) DNA molecules in host species cells, the source species DNA sequences will be in association with considerable amounts of host species DNA sequences and, possibly with considerable amounts of DNA sequences from artificial DNA constructs. The inventors of the present invention surprisingly have found that the source species DNA sequences maintain their self-inhibitory activity when in association with relatively large amounts of these non-source species DNA sequences. Thus elimination of host species DNA and/or DNA sequences from artificial constructs from the composition is not necessary. According to certain preferred embodiments, the composition of the invention therefore comprises non-source species DNA, wherein non-source species DNA is in excess of source species DNA, preferably such that the ratio of non-source species DNA : source species DNA is between 10:1 and 1000: 1, such as between 20:1 and 500:1, between 50:1 and 500:1, or between 100:1 and 500:1. Non-source species DNA comprises and preferably consists essentially of, host species DNA sequences and optionally DNA sequences from artificial DNA constructs, such as cloning and/or expression vectors, for example, selected from plasmids, BACs, YACs or artificial fungal chromosomes.

The source species preferably is selected such that inhibitory DNA sequences derived therefrom are inhibitory for a target species as discussed above. As is clear for the skilled person since the publication of WO2014/020624, inhibitory DNA sequences may be derived from a target species itself or from a phylogenetically similar species. In the context of the present invention, the source species thus may be selected from a target species or from a species phylogenetically similar to a target species.

Within the context of the present invention, a phylogenetically similar species is a species having a similar genome. The skilled person will understand that species that are phylogenetically closely related have a more similar genome than species that are phylogenetically distant. Phylogenetically similar thus means having a close phylogenetically relation. Phylogenetic similarity may thus be determined based on known phylogenetic relations. Thus according to certain preferred embodiments phylogenetically similar species are species within the same taxonomic order. Within a certain order, phylogenetically similar species are preferably from a same monophyletic group (clade), such as from a same family, a same subfamily, a same tribe, a same subtribe, a same genus. It is most preferred that phylogenetically similar species are from the same taxonomic family, such as a same subfamily, a same tribe, a same subtribe, a same genus.

In addition, techniques for determining genome similarity (or relatedness) are readily available. Genome similarity may for example be determined by determining the renaturation/reassociation kinetics of single stranded DNA (ssDNA) fragments of the genomes from both species. Alternatively, or in addition, denaturation (melting) of double stranded DNA (dsDNA) fragments renatured from mixtures of ssDNA fragments of the genomes from both species may be investigated. The latter technique allows for the definition of the melting temperature Tₘ, i.e. the temperature at which half of the DNA strands are in the ssDNA state and of the related T₅₀H. Approaches involving renaturation/denaturation kinetics and assessment of melting profiles were introduced in the early 70's (see de Ley et al. Eur J Biochem. 1970 Jan;12(1):133-42) for determining the relatedness of bacteria, but these approaches involving melting temperature profile analyses have also been used for determining the relatedness of eukaryotic species (see for example Sibley and Ahlquist, J Mol Evol (1984) 20:2-15). As is further known, since the publication of WO2014/020624, phylogenetic similarity of species can be determined on the basis whether inhibitory DNA fragments from one species are also inhibitory for another species. According to certain other embodiments a phylogenetically similar species is thus a species whereof DNA obtained by random fragmentation of extracted total DNA or by random fragment synthesis starting from total DNA is inhibiting for the target species. It will be clear for the skilled person that based on this functional definition phylogenetic can be determined with tests similar to those presented in WO2014/020624 and in the experiments attached herewith. Within the same taxonomic order a source species will also be phylogenetically similar to a target species, because DNA obtained from the source species by random fragmentation of extracted total DNA or by random fragment synthesis starting from total DNA is inhibiting for the target species.

The composition of the invention is intended for inhibiting a target species. Said target species may be identical to the source species or may be a species phylogenetically similar to the source species. To enhance the inhibition of a target species, the composition may be combined with the use of a number of other (natural) biocides, such as a (natural) pesticide, for example a fungicide, an insecticide, a nematocide, a miticide, an artropocide, a bactericide or an algaecide. The additional (natural) biocide may be a biocontrol agents, such as a microbial biocontrol agent, for example a bacterial biocontrol agent or a fungal biocontrol agent, in particular an entomopathogenic biocontrol agent. According to certain embodiments, the additional (natural) biocide is present in the composition. When the additional (natural) biocide is a microbial biocontrol agent, such as an entomopathogenic biocontrol agent, it is preferred that viable cells of the microbial biocontrol agent function as host cells comprising and replicating source species DNA. For example the source species may be selected from a target species or from a species phylogenetically similar to the target species wherein the target species is selected from Lepidoptera, in particular selected from the family Tortricidae, such as from the genus Choristoneura, in particular Choristoneura orae, Choristoneura fumiferana or Choristoneura freemani, or selected from the family Noctuidae, such as the genus Spodoptera, in particular Spodoptera frugiperda, Spodoptera litura, Spodoptera litoralis, Spodoptera cilium or Spodoptera ornithogalli, or selected from the family Pyralidae, such as from the genus Plodia or Ephestia, or from the Diptera, Coleoptera, or Hymenoptera, and most preferably is selected from the genus Choristoneura. The composition may then further comprises a biocontrol agent directed against the target species, such as a bacterial biocontrol agent, for example *Bacillus thuringiensis* or *Bacillus subtillus,* or a fungal biocontrol agent, for example an entomopathogenic fungus, such as *Beauveria bassiana*, *Isaria fumosorosea*, *Lecanicillium* spp., or a *Metarhizium* spp.. The biocontrol agent may be viable biomass of the bacterial biocontrol agent or the fungal biocontrol agent, preferably *Bacillus thuringiensis* biomass. Said viable biomass, such as *Bacillus thuringiensis* cells, may then also have source species DNA incorporated in a replicable form and thus functions as host species cells.

The composition can be administered to a target species by any suitable means, such as surface contacting, cytotropic administration, systemic administration by means of, for example, injection, ingestion or inhalation, or adsorption. The composition can be formulated in a form, for dry or liquid treatments, selected in the group consisting of dispersion, for example in form of aerosol, suspension, wettable or soluble powders, emulsions in water or other solvents, dispersible granules, suspensions of microcapsules, emulsifiable concentrates, fluid pastes, macro emulsions, oil dispersions, baits. Solvent systems comprising water or deep eutectic solvent (DES) systems such as natural deep eutectic solvent (NADES) systems may be used.

Determination of the concentration ranges wherein DNA of the composition of the invention is inhibitory for the target species is within the ambit of the knowledge of the skilled person. The skilled person will understand that the required concentration may depend on factors such as the potency of the DNA in the composition to inhibit the target species, the level of inhibition desired, whether or not an additional biocide is applied and/or the application route to the target species. For many applications, suitable concentrations may be in the range of 1-1500 ppm, such as 2-1300 ppm, 2-1000 ppm, 5-1000 ppm, 10-1000 ppm, 50-1000 ppm, 100-1000 ppm, 200-1000 ppm, 500-1000 ppm. For other applications higher concentrations may be desired.

As is clear from WO2014/020624, inhibitory DNA fragments may be used in medicine. Amongst others, WO2014/020624 shows inhibition of *Aspergillus niger,* which may cause infections such as otomycosis. Similarly the composition of the present invention comprising DNA fragments, or their precursors, may also be used in medicine for inhibiting *Aspergillus niger* or other parasitic and/or pathogenic organisms, such as nematode parasites. In the medical use of the compositions, the source species DNA is an inhibitory agent, or a precursor thereof, as when it is in unfragmented from it may be converted *in situ,* such as in the gastrointestinal tract, to a fragmented form. A further aspect of the invention is therefore, a composition of the invention for use in medicine, or in other words for use as a medicament. Medicinal uses in the context of the present invention relate to human medicine and/or veterinary medicine, preferably human medicine. Reference to an animal in the context of the present invention includes reference to a human, unless a different meaning would follow from the context wherein it is used. The composition may for example be used in medicine as an anti-pathogenic composition capable of inhibiting the spreading and/or growth of a pathogenic organism or even for killing a pathogenic organism. Pathogenic target organisms that may be inhibited with the composition of the invention are for example topical pathogens, intestinal pathogens, pulmonary pathogens or systemic pathogens. The pathogen may for example be a microbial pathogen, such as selected from bacteria, fungi or viruses, a parasite, such as selected from protists or selected from helminths, for example a nematode, tapeworm, fluke or roundworm. The medical use of the composition of the invention is in particular directed against pathogenic and/or parasitic target species presented above in the discussion of the composition of the invention, in particular topical pathogens and/or parasites associated with, skin, nail or mucosa.For use as a medicament the composition is in a pharmaceutically acceptable form.

The invention relates to the use of the composition of the invention as a product for inhibiting a target species. The target species may correspond to the source species or is a species phylogenetically similar to the source species. In the product the composition of the invention may be used as an active ingredient for inhibiting a target species. The intended inhibition of the target species may be in the product, for example to improve biological stability of the product, or alternatively the intended inhibition of the target species may be on a target surface or in a target area where the product is to be applied. The product may comprise further ingredients, including further ingredients inhibitory for parasitic, pathogenic and infesting species, such as one or more selected from biocides, herbicides, fungicides, insecticides, acaricides, nematocides, antiprotozoics, algaecides, bactericides. The product may be a medical product or may be a non-medical product. According to certain embodiments the product is a non-medical product such as an agronomical product, a personal health care product, or a sanitary product such as a soap or a detergent.

In certain preferred embodiments of the use of the composition, the source species DNA sequences may be in host species cells in chimeric DNA molecules. For some applications it is sufficient to present chimeric DNA molecules in hosts species cells, as they may be liberated from the host species cells by conditions to which the host species cells are subjected. For example where the product is a product for inhibiting a target species for which host species cells are a suitable food source, the chimeric DNA molecules comprising source species DNA sequences are liberated from the hosts species cells in the intestines of the target species, when they are eaten by the target species. In this process, source species DNA sequences may also be liberated from the chimeric DNA molecules. Also in a process of natural decay of the host species cells, chimeric DNA molecules contained in the host species cells may be liberated for the host species cells and source species DNA sequences may be liberated from the chimeric DNA molecules by fragmentaion. In an alternatively use of the invention, host species cells are disrupted by a cell disruption technique, such as a cell disruption technique that fragments DNA, and material from disrupted host species cells is used for inhibiting the target species. For this DNA may optionally be isolated from the disrupted host species cells with known methods. Alternatively, the disrupted cell mass may be used without further isolation of DNA for inhibiting the target species.

A further aspect of the invention relates to a method of producing a composition according to the invention. The method comprises the steps of:
- providing a source of DNA from the source species, optionally comprising fragmented source species DNA;
- providing cells of a number of species, the host species, differing from the source species;
- subjecting the host species cells with the source of DNA from the source species to conditions allowing the host species cells to take up source species DNA in a replicable form;
- isolating host species cells as a source of DNA inhibitory to the target species;
- optionally, isolating DNA inhibitory to the target species from the host species cells, in particular DNA containing source species DNA;
- optionally, fragmenting DNA content of host species cells, in particular DNA containing source species DNA.

In the first step, a source of DNA from the source species, optionally comprising fragmented DNA, is provided. The source of DNA from the source species may be any suitable source. For example cells of the source species may be used as such.

Alternatively, DNA released from source species cells may be used, for example DNA released form cells disrupted by a cell disruption technique, such as a cell disruption technique that fragments DNA. Released DNA may or may not be isolated. Isolated DNA may or may not be fragmented. Fragmented DNA from the source species may be presented as such or may be presented incorporated in artificial DNA constructs, such as in artificial plasmids, cosmids, artificial chromosomes, such as YACs or BACs or fungal artificial chromosomes or in a constructed chimeric DNA molecule in association with transposable elements.

In the second step of the method, the number of host species is provided. Based on what has been discussed in connection to the composition of the invention, the use of the invention and the common general knowledge available, the skilled person will know and understand how to provide suitable host species cells.

In the third step, the host species cells with the source of DNA from the source species are subjected to conditions allowing the host species cells to take up source species DNA in a replicable form. By taking up source species DNA in a replicable form, the host species cells will replicate the source species DNA with its DNA replication machinery. Thus the conditions must be such that the source species DNA, as provided, is incorporated in host species cells such that it is functionally available for the DNA replication machinery of the host species cells to be replicated. The skilled person will be able to select suitable conditions for allowing the host species cells to take up source species DNA in a replicable form.

For example when source species DNA is provided incorporated in artificial DNA constructs such as in plasmids, cosmids, artificial chromosomes or in a constructed chimeric DNA molecule in association with transposable elements, the skilled person will know how to condition the host species cells to make them competent to effectively take up these artificial DNA constructs such that they may be effectively replicated.

In case the source species DNA is provided as cells of the source species. The skilled person will know that the required conditions must allow the source species cells to release the source species DNA and the source species DNA to fragment. It has been found that in the process of litter decomposition, the conditions are such that DNA from plant material in the litter may be transferred to the soil microbiome. Based on these findings the skilled person will expect that also DNA from non-plant sources included in litter or a similar decomposing biological material, may be incorporated in the soil micobiome. Suitable conditions may thus be conditions of litter decomposition, in particular where the host species is a soil microorganism such as selected from Alphaproteobacteria spp., Betaproteobacteria spp., Deltaproteobacteria spp., Actinobacteria spp., Thermoleophilia spp., Rubrobacteria spp., Chloracidobacteria spp., Acidobacteria spp., or Solibacteres spp..

In case the source species DNA is provided as DNA released from source species cells, the skilled person will know that the released source DNA may be taken up with natural processes by suitable cells of the hosts species or via forced methods such as biolistics (or gene shotgun).

The fourth, step comprises the step of isolating host species cells. From the whole of this description, including the examples, the skilled person will understand that the isolation of host species cells is not required for all applications. Where isolation of host species cells is required or desired, the skilled person will have knowledge about suitable techniques for isolating host species cells.

The fifth, step is optional and comprises the step of isolating DNA from host species cells. From the whole of this description, including the examples, the skilled person will understand that the isolation of DNA from host species cells is not required for all applications. Where isolation of DNA from host species cells is required or desired, the skilled person will have knowledge about suitable techniques for isolating DNA from host species cells.

The sixth, step is a further optional step and comprises the step of fragmenting DNA of host species cells. From the whole of this description, including the examples, the skilled person will understand that fragmentation of DNA from host species cells is not required for all applications. Where fragmentation of host species DNA is required or desired, the skilled person will have knowledge about suitable techniques for fragmenting DNA from host species cells. In case DNA from host species cells is fragmented, it is preferred that chimeric DNA molecules are fragmented. DNA fragmentation by random fragmentation techniques are preferably used.

The invention will now be further exemplified by the following non-limiting experiments.

### EXPERIMENTS

### EXPERIMENT 1

### Arabidopsis thaliana libraries

Commercial microbial genomic libraries of *Arabidopsis thaliana* were purchased from TAIR (https://www.arabidopsis.org/) engineered in both yeast and *E. coli.*

The YAC library CD4-21P was a pooled yeast artificial chromosome (YAC) genomic library in pYAC4 vector; DNA isolated from nuclei of Columbia plants; original library consisted of 1,152 clones; average insert size = 420 kb; 21% of clones carry chloroplast DNA; library consists of 81 pools; cloning enzymes: EcoRI and EcoRI.

The BAC library CD4-21P binary cosmid genomic library in POCA18-hyg vector; generated from Wassilewskija (Ws) genomic DNA fragments created by partial digestion with TaqI; represents ten genome equivalents; vector carries a hygromycin-resistance gene under the control of the CaMV 35S promoter; cloning enzymes: ClaI and ClaI.

Each library was used to obtain a microbial solution prepared as follows:

### YAC library CD4-21P:

Each of the 80 stocks of the library were inoculated in a tube containing YPD medium (1 % yeast extract, 2 % bactopeptone, 1 % glucose) and incubated at 28 °C for 48 h. After incubation, the tubes were pooled, the cell suspensions centrifuged (4000 rpm, 5 min), and resuspended in 10 ml NaCl 0,9% (pooled yeast suspension). Then, the pooled YAC culture was performed in a bench stirred reactor (New Brunswick) containing 1 L of YPD medium, at 28 °C, 200 rpm, 1 vvm aeration rate. The reactor was inoculated with an adequate aliquot of the pooled yeast suspension to give an initial O.D.₅₉₀ = 0.1.

After 48 h, the yeast biomass was collected, washed and resuspended in distilled water to achieve a final concentration of 50 mg dry weight of cells per ml.

For the yeast cells to be used as control, the same procedure was followed, but using as inoculum for the reactor a pre-culture of a strain of *S. cerevisiae* CEN.PK113-7D in YPD medium. Also, in this case, the produced biomass was collected, washed and resuspended in distilled water to the same cell density reported above.

### BAC library CD4-21P:

Each of the 80 stocks of the library were inoculated in a tube containing Tryptone Soya Broth (TSB) with 50 mg ml⁻¹ hygromicin and incubated at 37 °C for 48 h. After incubation, the tubes were pooled, the cell suspensions centrifuged (10,000 rpm, 10 min), and resuspended in 10 ml NaCl 0,9% (pooled bacterial suspension). Then, the culture of the bacterial library was performed in a bench stirred reactor (New Brunswick) containing 1 L of TSB medium with hygromycin, at 37 °C, 200 rpm, 1 vvm aeration rate. The reactor was inoculated with an adequate aliquot of the pooled bacterial suspension to give an initial O.D.₅₉₀ = 0.01. After 48 h, the bacterial biomass was collected, washed and resuspended in distilled water to achieve a cell density of 50 mg dry weight of cells per ml.

For the bacterial cells to be used as control, the same procedure was followed, but using as inoculum for the reactor a pre-culture of *Escherichia coli* ATCC 10536 in TSB. Also, in this case, the produced biomass was collected, washed and resuspended in distilled water to the same cell density reported above.

### Inhibition tests

The microbial solutions of the two libraries were sonicated and the resulting cell homogenates were incubated under aerobic conditions at room temperature for 15 days compensating the water evaporation by addition of distilled water. The, different aliquots of the homogenates were diluted 1:1, 1:10, and 1:100 with distilled water and used to wet filter paper in petri dishes with three replicates with 10 plants for each concentration level. *Arabidopsis thaliana* seedlings were prepared by germinating seeds in petri dishes with standard nutrient solution on filter paper. Healthy seedlings of at least one week of age were selected of similar size to be used for the inhibition test. The experiment was performed by exposing the seedlings to the different solutions in the Petri dishes (10 seeds per dish, 10 dishes per treatment). A control was assessed by seedlings maintained for the duration of the experiments only with distilled water. The treatments consisted in the exposure to either the library or to the corresponding microbial solution without genomic library. Survival of seedlings was assessed after 10 days by counting number of living plants. The experiment was done in a growth chamber maintained at optimal level of temperature and air humidity.

### Results

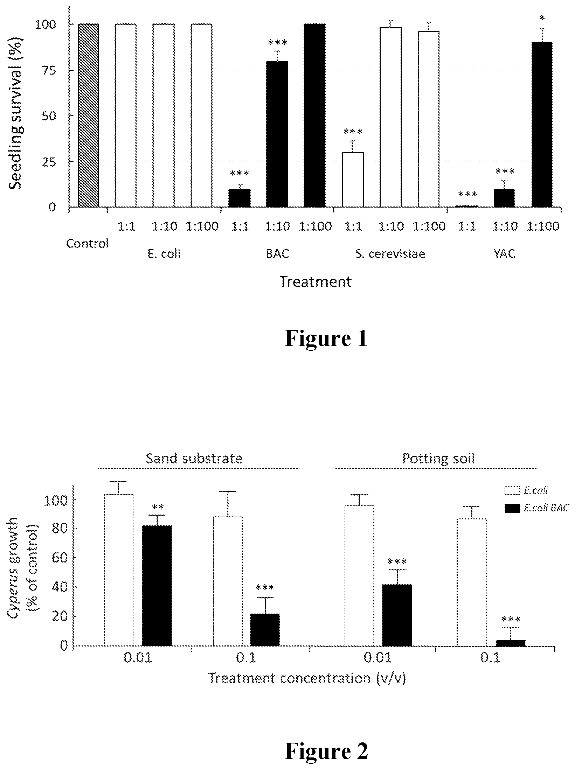

The results of the inhibition tests demonstrated the inhibitory effects by mixtures of heterologous and self-DNA by testing microbial libraries (BAC and YAC) of genomes of *Arabidopsis thaliana* on conspecific seedling survival. Indeed, seedling survival was significantly lower under exposure to YAC and BAC libraries of genomic self-DNA compared to both the control treated with distilled water, and the corresponding microbial solution including heterologous DNA but not the self-DNA genomic library (Fig. 1). This pattern was consistent across the three levels of treatment dilution. Moreover, treatments containing heterologous DNA but not self-DNA generally did not affect seedling survival, which did not differ from that of the control, with the exception of the *S. cerevisiae* material, which showed, non-specific inhibitory effect at the highest concentration level.

### EXPERIMENT 2

### Cyperus aesculentus library

A genomic BAC library of *Cyperus aesculentus* was constructed by Bio S&T (Montreal, Canada) in *E. coli* EPI300 using the vector pSmart-HindIII (Chloramphenicol resistance). The library was pooled in 1 x 96-well plate, with each well containing 123 primary clones, for a total of 11,808 clones (> 2x genome coverage). The average insert size (110 Kb) was determined using tested random clones, digested with NotI. The library was stored at -80 °C.

The library was used to obtain a microbial solution prepared as follows:
For amplification, each well was inoculated from frozen stock in a tube containing Tryptone Soya Broth (TSB) with Chloramphenicol at 12.5 mg/L and incubated at 37 °C for 48 h. After incubation, the 96 tubes were pooled, the cell suspensions centrifuged (10,000 rpm, 10 min), and resuspended in 10 ml NaCl 0,9% (pooled bacterial suspension). Then, the culture of the bacterial library was performed in a bench stirred reactor (New Brunswick) containing 1 L of TSB medium with Chloramphenicol, at 37 °C, 200 rpm, 1 vvm aeration rate. The reactor was inoculated with an adequate aliquot of the pooled bacterial suspension to give an initial O.D.₅₉₀ = 0.01. After 48 h, the bacterial biomass was collected, washed and resuspended in distilled water to achieve a cell density of 50 mg dry weight of cells per ml.

For the bacterial cells to be used as control, the same procedure was followed, but using as inoculum for the reactor a pre-culture of *Escherichia coli* EPI300 in TSB. Also, in this case, the produced biomass was collected, washed and resuspended in distilled water to the same cell density reported above.

### Inhibition tests

The microbial solutions were sonicated and the resulting cell homogenates were incubated under aerobic conditions at room temperature for 15 days compensating the water evaporation by addition of distilled water. Then, different aliquots of the homogenates were diluted 1:10 and 1:100 v/v with each of two different substrates for plant growth (quartz sand and standard potting soil). *Cyperus aesculentus* seedlings were prepared by germinating seeds in petri dishes with standard nutrient solution on filter paper. Healthy seedlings of similar size, aged at least 10 days, were selected to be used for the inhibition test. The experiment was performed by exposing the seedlings to the different substrate mixtures in small plastic pots of 15 mL volume (1 seedling per pot, 20 pots per treatment). Treatments included factorial combinations of substrate type (two-levels, either sand or pot soil), *E. coli* material type (two levels, either with or without the BAC library), and solution concentration (two levels, either 1:10 or 1:100). Pots containing unmixed substrate were considered as controls (10 pots for each substrate type). *C. aesculentus* growth in controlled conditions (T day/night of 22/16 °C, photoperiod 16:8 hrs, ambient air humidity, daily watering to pot field capacity) was assessed 10 days since seedling potting, by destructive sampling of plant material, followed by oven-drying (60° C x 48 h) and weighing of total biomass.

### Results

The results demonstrated the inhibitory effects of BAC genomic library of *Cyperus aesculentum* on conspecific seedling growth. Indeed, seedling biomass was significantly lower under exposure to BAC library of genomic self-DNA compared to both the control treated with distilled water, and the corresponding microbial material including heterologous DNA but not the self-DNA genomic library (Fig. 2). The inhibitory effect was dose-dependent, consistent across the two levels of treatment concentration. Treatments containing heterologous DNA but not self-DNA did not affect seedling growth, which did not differ from that of the control.

### EXPERIMENT 3

### Caenorhabtidis elegans library

A fosmid library of C. *elegans* was purchased (commercially available) at Source BioScience (http://www.sourcebioscience.com/).The library included 41 x 384 plates with a total of 15,744 clones. The large insert (average size 43.3 kb) library was constructed in E. coli EPI300-T1R using the fosmid CopyControl vector pCC1FOS (Chloramphenicol resistance).

Two E. coli strains were used: OP50 and EPI300-T1R. E. coli strains were grown in LB growth medium. Chloramphenicol at the final concentration of 12.5 µg/ml, and arabinose at the final concentration of 0.01% weight/volume were added to the growth medium where required. *C*. *elegans* strain was the wild type Bristol (N2). The C. *elegans* strain was maintained at 20°C on NGM agar plates (without arabinose) spread with E. coli OP50 as food source. E. coli EPI300-T1R and EPI300-T1R transformed with the pCC1FOS based library were used as a food source on NGM plates (with arabinose) as specified.

As for fosmids amplification, each microtiter plate, containing 384 library clones, was inoculated from frozen stocks on LB agar plates containing Chloramphenicol (150mm Petri dishes). The resulting 41 plates were incubated at 37°C overnight. After incubation bacteria were collected in 12 ml of LB broth containing chloramphenicol, spread on each plate with the help of a spatula. The resulting bacterial mixture was transferred in 15 ml culture tubes (41 tubes). 100 µl were inoculated in 4 ml LB broth containing chloramphenicol and arabinose and incubated overnight at 37°C (41 tubes). The next day 100 µl of the bacterial cultures were transferred in 2 ml LB broth containing chloramphenicol and arabinose and incubated overnight at 37°C (41 tubes).

### Inhibition tests

The resulting 41 bacterial cultures were pooled and 20 µl of these pooled bacteria were used on each NGM plate containing arabinose (35 mm). Untransformed E. coli EPI300-T1R was used as negative control. In this case bacteria were grown with the same procedure followed for fosmids amplification but chloramphenicol was omitted. For screening generation I, a single L4-stage *C*. *elegans* worm was transferred on each NGM plate containing arabinose and inoculated with bacteria, and kept at 20°C. This worm was transferred every 12 hours onto a fresh identical plate to allow deposition of all the eggs (3 days).

The following parameters were monitored: embryonic lethality (ratio of unviable eggs to laid eggs) and aberrant phenotypes (ratio of aberrant phenotypes to the hatched eggs). The same parameters were also monitored for screening generation II, for which a single, not defective L4-stage *C*. *elegans* worm from generation I was transferred on each NGM plate containing arabinose and inoculated with bacteria, and treated as described above for screening generation I.

Inhibition with a fraction of the *C*. *elegans* genome was also tested. For this, worms were fed with cloneA01(WRM0610aA01) (Chromosome I; clone start: 13249863 - clone end: 13287543 [size 37680]) of the *C*. *elegans* library, cloneB01(WRM0610cA02) (Chromosome X; clone start: 12671842 - clone end: 12705840 [size 33998]) of the *C*. *elegans* library and a combination of cloneA01 and cloneB01. Embryonic lethality (ratio of unviable eggs to laid eggs) and aberrant phenotypes (ratio of aberrant phenotypes to the hatched eggs) were tested in the first generation offspring.

### Results

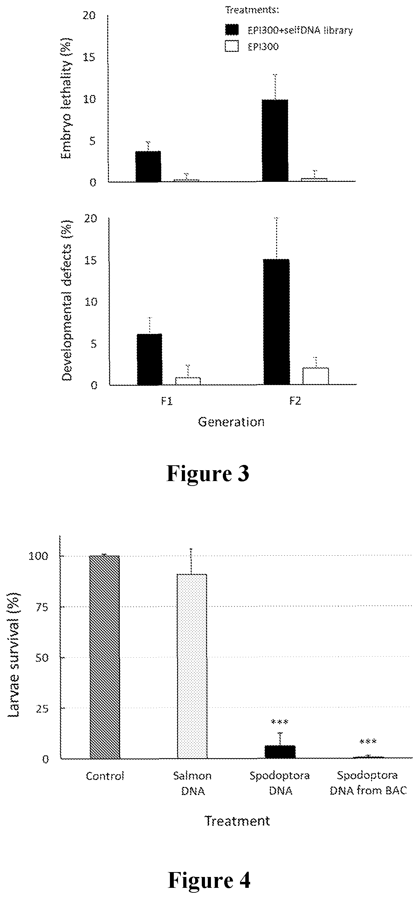

In the experiment, eleven worms fed on EPI300-T1R containing the whole *C*. *elegans* fosmid Library and twelve worms fed on untransformed EPI300-T1R were analyzed in generation I screening (2574 and 3055 eggs respectively). Worms fed on EPI300-T1R containing the whole *C*. *elegans* fosmid Library showed an increase of embryonic lethality, significantly different from that of worms fed on EPI300-T1R (3.85% and 0.82% respectively, p < 0.0001). Ratio of males to the hatched eggs did not differ between treatments, as males were absent in both progenies. A significant higher frequency (6.32%) of developmental defects was observed in progeny of worms fed on EPI300-T1R containing the whole *C*. *elegans* Fosmid Library compared to that of worms fed on EPI300-T1R (p < 0.0001, Fig. 3).

Notably, negative effects by the whole *C*. *elegans* fosmid Library were exacerbated in generation II, compared to generation I (Fig. 3).

The results of the inhibition by a fraction of the genome are presented in Table 2 below.

| | CloneA01⁽¹⁾ | CloneB01⁽²⁾ | CloneA01 + Clone B01⁽³⁾ |
|---|---|---|---|
| Embryonic lethality | 0.29 % | 0.77% | 4.8% |
| Aberrant phenotype | 1.09% | 0.2% | 1.81% |

| | | | |
|---|---|---|---|
| (1) 5 worms fed, analysis on 1378 eggs; (2) 5 worms fed, analysis on 1299 eggs; (3) 12 worms fed, analysis on 2726 eggs. % values are significant differences compared to control. | | | |

The results show that a small fraction of the genome produces significant effects on both embryo lethality and appearance of aberrant phenotypes. The combined clones give an increased effect (higher than additional). It may be expected that by combining with further clones, the inhibitory effects can be progressively increased.

### EXPERIMENT 4

### Spodoptera littoralis library

A genomic BAC library of *S. littoralis* (cotton leafworm) was constructed in *E. coli* DH10B using the vector CopyControl pCC1BACH (BamH I Hind III, EcoR I Cloning Ready Vector - 8128 bp) at the Hind III site, after extraction and digestion (restriction enzyme Hind III) of high molecular weight genomic DNA from *S. littoralis* larvae grown in laboratory and then frozen. Clones were grown on plates containing Luria Bertani (LB) + chloramphenicol solid substrate and selected. Clones were screened and picked, expression induced to a high-copy number, then transferred to wells in a 96-deep well plate. Each well contained 290 primary clones, for a total of 27,840 clones (> 10x genome coverage). The average insert size (132 Kb) was determined using tested random clones, digested with NotI. An empty vector control was also prepared and grown in the same conditions of the library vector. The library and the empty vector were stored at -80 °C.

The library was used to produce plasmid DNA as follows: a single sterile toothpick was immersed and wetted in each single well culture, then transferred and used to inoculate to a 2 mL microcentrifuge tube containing 1 ml of LB with chloramphenicol (12.5 µg/ml). Once inoculated, the microfuge cultures were incubated at 37°C, in orbital agitation, overnight. All 96 starter cultures (96 ml) were pooled together, to obtain a starter inoculum representative of the entire library. Ten liters of LB with chloramphenicol were prepared, divided among glass Erlenmeyer flasks and sterilized. Each substrate recipient was inoculated with the starter inoculum, incubated at 37°C, in orbital agitation, overnight.

For the extraction of plasmid DNA from both the library and the empty vector control, the QIAGEN Plasmid Giga Kit was used, following manufacturer's instructions.

The DNA concentrations obtained were 688 ng/µl for the library, in ca. 800 µl volume (Total yield DNA=550 µg), and 268 ng/µl for the empty vector.

### Inhibition tests

Larvae of *Spodoptera littoralis* at the third-instar stage were kept in controlled conditions (T 22-24 °C, photoperiod 16:8 hrs) in 15-cm Petri dishes and fed as previously described (Bergomaz and Boppré, 1986). Treatments included solutions of DNA fragments, obtained from salmon (*Salmo salar*) and *S. littoralis* by extraction and sonication as described in Mazzoleni et al. (2015b), and a mixture of self- and heterologous DNA obtained by extraction from the BAC library as described above.

Each of the three treatment solutions were diluted at a DNA concentration of 200 ng/µl, and 4 mL of each solution was included in the daily diet of the larvae. Twenty replicates for each treatment (Petri dishes, each with 30 larvae) were maintained in controlled conditions for 20 days since the start of the growth experiment. Control replicates (N = 10) were also maintained in the same conditions, and fed with the standard, untreated diet. Larvae survival for all treatments was assessed at the end of the 20 days growth period.

### Results

Results of tests in EXPERIMENT 4 showed that the survival of *Spodoptera littoralis* larvae is significantly affected by exposure to self-DNA by ingestion, both in the presence and in absence of heterologous DNA (i.e. DNA of the microbial strain used as the BAC library vector) in the feeding pellet. Indeed, in these two cases survival rates of 5% and 0% were observed after 20 days (Fig. 4). Oppositely, in the case of feeding pellet containing exclusively heterologous DNA (i.e. salmon DNA), no significant survival reduction was observed compared to the untreated control, demonstrating that heterologous DNA alone does not induce inhibitory effects on the larvae (Fig. 4).

### EXPERIMENT 5

In a confirmatory experiment, to further confirm the findings of the above experiments that fragmented self-DNA is inhibitory for individuals of a species when presented in association with heterologous DNA (e.g. DNA of a host organism used for DNA amplification), inhibition of *Drosophila melanogaster* by self-DNA obtained with amplification in yeast, a suitable food source for *D. melanogaster,* was tested.

### Self-DNA amplification

A culture of *Drosophila Melanogaster* was obtained with a standard rearing method. Yeast cells containing fragments of *D. melanogaster* DNA were engineered at Bio S&T (Montreal, Canada). For this total DNA isolated from the indivuals of the *D*. *melanogaster* culture was partially digested using Sau3AI. The digested DNA fragments were cloned in the pGADT7 vector using the BamHI cloning site. *E. coli* DH10B host cells were transformed with the pGADT7 vectors containing the cloned *Drosophila Melanogaster* fragments and the resulting library was amplified on agar medium by overnight culture. About 120,000 original clones were plated on large Petri dishes (total of 10 dishes) on a semi-solid medium. Colonies were then washed and collected into 10 tubes. A maxi-prep was done to isolate library DNA.

Isolated DNA from the *E. coli* library was then used to transform *S. cerevisiae* Y187 host cells. Library are amplified on agar medium by overnight culture and plating about 120,000 original clones per large Petri dish (total of 10 dishes) on a semi-solid medium. Colonies are then washed and collected into 10 tubes. Insert size determination was done on 10 randomly chosen clones by gel chromatography showing an average size of 4k with a range of 1-6kb.

### Inhibition test

The yeast library was used as component of a standard feeding diet of *Drosophila* (Sang (1978)) from hatching till pupa stage. The treatments consisted of: Self-DNA treatment: feeding with yeast library; Control 1: feeding with same yeast strain without vector as control; Control 2: feeding with the same yeast strain with empty vector;. The results are presented in table 3 below.

**Table 3. Inhibition of D. Melanogaster early life stages by fragmented self-DNA in yeast library**

| | Stage duration (days) | | Mortality (%) | |
|---|---|---|---|---|
| | Larvae (3^{rd} instar) | Pupa | Larvae (3^{rd} instar) | Pupa |
| Control 1 | 9 | 6 | *6 (n=100)* | *0* (*n=94*) |
| Yeast library | 18 | 13 | 40 (n=100) | 50 (n=60) |

The results showed a significant inhibition for larvae fed with the yeast library containing Drosophila DNA. A significant elongation of time of development (double time of control) and mortality (40% at third instar phase and 83% of pupa) were observed during larval growth. No differences were observed between control 1 with standard diet and control 2 with yeast with empty library vector, thus demonstrating that the inhibition (determined on the level of life stage duration time and mortality) were due to the *Drosophila* DNA insertion in the yeast library.

### EXPERIMENT 6

### Inhibition of Pinus halepensis and Populus nigra

On the basis of the effective inhibition of a target species by (a fraction of) self-DNA fragments incorporated in artificial DNA constructs replicated in a host organism, it can be expected that self-DNA incorporated in the natural genome of a host species similarly will be effective in inhibiting the target species. Lateral gene transfer is known to occur widely in nature, including in soil microbiomes.

To confirm the inhibition of a target species by self-DNA incorporated in the natural genome of a host species, in this experiment soil microorganisms, two different litter materials (leaves of *Pinus halepensis* and *Populus nigra)* were inoculated with soil microorganisms from soil of an oak forest were the two species were absent. The decomposed litter was used for an inhibition test as in Mazzoleni et al. 2015. The decomposed litter material showing the conspecific inhibition was used for metagenomic analysis of the microbiome DNA to confirm the integration of plant DNA in soil microbiome DNA.

In detail, fresh leaves of *P. halepensis* and *P. nigra* were collected from the field and used in the decomposition experiment in the following way. Freshly abscised leaves were collected in natural communities by placing nets under randomly selected plants (n > 20), dried at room temperature in a ventilated chamber until a constant weight was reached and then stored at room temperature. The decomposition experiment was carried out in a growth chamber with optimal water availability and temperature conditions: the litter was watered every 7 d to holding capacity with distilled water and the temperature was 18 ± 2°C at night and 24 ± 2°C during the day. Dry leaf litter (100 g for each species in three replicates) was placed inside plastic trays (size 30 x 50 x 50 cm). A microbial inoculum, collected from an oak forest were the two species were absent, was prepared and distributed according to Bonanomi et al. (2011). Litters were collected after 120 days inoculation and dried in paper bags (at 40°C until a constant weight was reached).

In order to confirm the species-specific inhibitory effect of decomposed litter materials, bioassay tests were performed as follows: an 8-cm-wide sterile filter paper strip was placed in square Petri dishes (size 12 x 12 x 1.5 cm). Pre-germinated seeds of each species (five for each dish) were placed at the top of the paper strip previously amended with the different decomposed litter materials. Root observation plates were placed at 45° and covered with opaque sheets. Root length was assessed after 28 days.

In order to separate the microbial populations from the decomposed material, 1 g of litter sample was placed in 5ml tubes. 3 ml of phosphate buffered saline (PBS) were added to each sample. Samples were homogenized by vortexing for 60 seconds and subsequently centrifuged at 10000 rpm for 1 minute to separate microbial cells from litter debris. After centrifugation surnatants of separated microbial cells were transferred to new 2 ml tubes. Following pooling phase, DNA was extracted from the microbial cells by CTAB method. At the end of the extraction step, the samples were treated with Polivinilpirrolidone (PVP) to remove phenols to avoid possible inhibition for PCR. Extracted DNA was furtherly purified with Ethanol than dried in microbiological laminar air flow chamber and eluted in nuclease-free PCR grade water.

Metagenomics analysis of the decomposed litters was performed by standard shotgun sequencing techniques. Paired-end reads from metagenomics were then analyzed, according to the bioinformatics procedure described below, to find recombinant fragments belonging to both microbial species and the DNA from the plant species of the decomposing litter.

In order to find possible recombinant DNA fragments belonging to both the abovementioned plants and species of the corresponding microbiomes, the following procedure was followed. Paired-end reads from metagenomics experiments were merged using the BBmerged function of BBmap (Bushnell 2016), applying the kmers option to reduce false positives (extend2=20, iterations=5). The paired-end that were not merged, are fused using the fuse.sh function of BBmap (Bushnell 2016), inserting 0N between the forward and the reverse read. Merged and fused reads were then aligned versus the host reference genome, or a related species if the genome was not available, using BLASTn (Camacho et al. 2009). The following reference genomes were used: *Pinus taeda* (1760464 scaffolds ~22GB) and *Populus trichocarpa* (1694 scaffolds, ~500MB). All the reads that had at least one hit with the host genome were considered as matching. The reads marching the host genome were independently aligned versus the NCBI's non redundant database using BLASTn (Camacho et al. 2009) in order to confirm the match with the reference species.

### Results

Bioassays experiments confirmed the species-specific inhibitory effect of decomposed litter for *P. halepensis* and *P. nigra.* Root growth of germinated seeds of *P. halepensis* was 50% less on their own litter compared to growth on *P. nigra* litter. *P. nigra* root growth was 70% less on their own decomposed litter compared to litter of *P. halepensis.*

Analysis of metagenomics data of the decomposed litters clearly showed the presence of several recombinant DNA fragments between plant species and microbial organisms. Starting from 44,431,532 merged reads, 108,288 reads matched the *Pinus taeda* genome scaffolds. The 108,288 reads were then compared versus the NCBI nucleotide database (NT) and 100% of the reads found at least one match in the NT database with 41,533 unique IDs. Among the unique IDs, we selected a random subset of 10,000 IDs to get the detailed taxon annotation. From the total number of matching sequences (108,288), the annotations were confirmed for a subset of 29,149 sequences, where 4,595 sequences were confirmed to match with *P. taeda* sequences in NT. Among these 4,595 sequences, 1,359 had a coverage lower than 80%, meaning that the sequence can be considered as a recombinant sequence between pine and a microbial species. To confirm the specificity of the pine genome uptake by the microbes in the pine litter, the matches with *P. trichocarpa* were also checked and only 139 were found.

Similar results are found in the case of the *Populus nigra* litter. The annotation results are summarized in Table 4 and 5.

The presented analysis by shotgun approach clearly confirms the enrichment in the metagenomics data of fragments with partial similarity with the reference genome of the decomposing plant litter. In other words, the results show the specific inclusion of fragments of DNA from the litter substrate, thus creating a microbiome which is marked by the plant showing the inhibitory effect.

**Table 4. Results of the metagenomics analysis on P. halepensis litter.**

| *P. taeda* reads versus *P. taeda* genome | Annotated sequences based on a sample of 10,000 random unique IDs | Sequences which were confirmed to match *P. taeda* annotations | Putative recombinants: confirmed *P. taeda* with coverage <80% | Sequences which were confirmed to match *P*. *trichocarpa* annotations |
|---|---|---|---|---|
| 108,288 | 29,149 | 4,595 | 1,359 | 139 |

**Table 5. Results of the metagenomics analysis on P. nigra litter.**

| *P. trichocarpa* reads versus *P*. *trichocarpa* genome | Annotated sequences | Sequences which were confirmed to match *P*. *trichocarpa* annotations | Putative recombinants: confirmed *P. trichocarpa* with coverage <80% | Sequences which were confirmed to match *P. taeda* annotations |
|---|---|---|---|---|
| 80,355 | 80,355 | 16,281 | 3,206 | 39 |

To confirm these remarkable findings, the experiment was repeated and the analysis was extended. Collection of the leaves, production of the litters and handling of the materials was as described above. The litter materials were incubated and decomposed in optimal conditions for 120 days and samples were collected after 30 days and at the end of the experiment. The fresh litter and the decomposed materials for 30 and 120 days were used for DNA extraction with standard commercial kits as described by Mazzoleni et al. (2015) and the extracted DNA was analysed. We compared reads obtained from a shotgun sequencing approach on litters (0, 30, 120 days of decomposition) from host plant species (Pinus and Populus) versus the indicated reference genomes.

The bioinformatic analysis was performed according to the following procedure: Paired-end reads from metagenomics experiments are merged using the BBmerged function of BBmap (Bushnell (2016)), applying the kmers option to reduce false positives (extend2=20, iterations=5).

Merged reads are then aligned versus the host genome using BLASTn (Camacho et al. (2009) ). All the reads that have at least one hit where lister as total reads having a match and they are listed in groups according to their coverage. Possible recombinant fragments are those matching from 20 to 80 or 90 % coverage. Filtering at 90% identity is also considered. Further details on the bioinformatic procedure can be found in Bushnell(2016) and Camacho et al. (2009).

The results are presented in table 6. They show that the frequency of whole DNA fragments of the plants recognized in the samples decreases in time, proving that the amount of free plant DNA in the litter decreases as the decomposition proceeds. On the other hand, the number of partial fragments matching with the plant genome (meaning that they are fragments composed in part of plant DNA and part of DNA of something else) increases as the decomposition proceeds (Table 7), with a peak after 120 days. It is important to note that litter materials decomposed for 120 days are the one showing the biggest inhibitory effect on the germination of seeds of the same species (i.e. *Pinus* and *Populus).*

Data were analysed in terms of number of sequencing matching (Table 6) in each sample and number of relative coverage of each read from portion of the genome (Table 7). Partial covered reads are assumed to reflect chimeras in the read sampling.

**Table 6. Frequency of matches over blasted reads**

| | | | | |
|---|---|---|---|---|
| | **PINUS** | | **POPULUS** | |
| | **No filter** | **Filter at 90% ID** | **No filter** | **Filter at 90% ID** |
| 0 DAY | 0,8951 | 0,6714 | 0,782 | 0,834 |
| 30 DAY | 0,1456 | 0,1090 | 0,002 | 0,001 |
| 120 DAY | 0,0016 | 0,0010 | 0,001 | 0,001 |

Table 6 shows that considering the frequency of matches from a blast search analysis versus the Pinus and Populus genomes, respectively, produces higher numbers in a 0 Day litter than in a 120 day litter. This means that the similarity versus the host plant is reduced due to sequences decay.

**Table 7. Relative frequency of reads per coverage**

| | | **PINUS** | | **POPULUS** | |
|---|---|---|---|---|---|
| | | relative frequencies per coverage no filter | relative frequencies per coverage at 90% identity | relative frequencies per coverage no filter | relative frequencies per coverage at 90% identity |
| 99<=coverage<=100% | **0 DAY** | 0,51 | 0,57 | 0,65 | 0,58 |
| | **30 DAY** | 0,49 | 0,55 | 0,33 | 0,42 |
| | **120 DAY** | 0,22 | 0,24 | 0,28 | 0,36 |
| | **0 DAY** | 0,13 | 0,10 | 0,14 | 0,23 |
| | **30 DAY** | 0,14 | 0,12 | 0,39 | 0,36 |
| | **120 DAY** | 0,44 | 0,40 | 0,41 | 0,36 |

Table 7 shows the relative decrease in high coverage of the reads when compared versus the host plant (decrease of 99 to 100 % coverage values) and increase of partial covered sequences (20 to 90% coverage) according to the litter aging.

Similarity versus the host genome was also investigated at 90% identity to discard possibile unreliable similarities versus the host plant. Decreased coverage indicates the presence of chimeras in the reads sequenced from the litter.

A very interesting point is that, on one hand, the number of matches with the plant genomes shows a very high decrease in the fast decomposing litter of Populus, whereas the slower decomposition of the Pinus litter corresponds to a much slower disappearance of the pine sequences. On the other hand, both species materials show a constant increase of number of mixed fragments in which the species can be still recognized, reflecting the natural incorporation into the microbial organisms.

These results support formation of natural libraries similar towhat is described further in this application for the single species culture of microalgae (see below). In this case the phenomenon of natural library formation occurs in a natural soil microbiome which becomes enriched with the DNA of the plant litter of its surroundings, thus producing the species specific inhibitory effect for the plant(s) producing the litter.

### EXPERIMENT 7

### Arthrospira platensis natural library

To further confirm the inhibition of a target species by self-DNA incorporated in the natural genome of a host species, the natural uptake of DNA was also induced by incubating *A. platensis,* a species belonging to cyanobacteria, with DNA extracted from *Arabidopsis thaliana* and the transformed *A. platensis* cells were used for inhibition of *A. thaliana.*

Before the incubation the pure culture of the cyanobacterium was maintained in controlled conditions (T 22-24 °C, photoperiod 12:12 hrs, irradiance 20 µmol photons m⁻² s⁻¹) on Zarrouk's growth medium without insufflation for at least 4 hours before the incubation.

The *Arabidopsis thaliana* DNA for the incubation was prepared by standard methods for whole genome extraction from fresh leaves and randomly fragmented by sonication bursts (details on the protocol reported by Mazzoleni et al. (2015a). The samples have been sonicated in a VC 505 SONICS Ultrasonic processor with a microtips immersed in a 1,5 ml microtubes with cold water in the water bath for 30 minutes with 45sec/15 sec on/off cycles at 20% of the amplitude.

### Incubation of Arthrospira platensis with Arabidopsis thaliana DNA

Before the incubation, *A. platensis* was cultivated in a Mg⁺⁺ deficient growth medium in order to decrease the intracellular and extracellular DNAse activity of the cyanobacteria and to facilitate the persistence and incorporation of the foreign DNA (Cao et al., 1999) During the incubation, 300 µl of concentrated *A. platensis* cells (up to 5 × 10⁷ cells obtained by gentle centrifugation) were added to a 15 mL centrifuge tube containing 300 mg of glass beads (diameter of 500 µ). Then, 100 µL of *A. thaliana* DNA previously fragmented by sonication bursts and 100 µL of Mg⁺⁺ deficient medium were added in the incubation tube, followed by agitation in a vortex mixer at top speed for 15 S.

The sample volume was increased to 2.5 ml with Zarrouk's Mg⁺⁺ deficient medium in the same tube and incubated in an oscillator under LD illumination cycles for 24 hours. Then the volume was progressively increased up to 10 l with the complete Zarrouk's Medium in a photo-bioreactor achieving a cell density of 1 g dry weight of cells per l. After 90 days, the biomass was centrifuged and collected obtaining a pellet of material with a cell density of 100 g dry weight of cells per l.

### Inhibition tests

The cyanobacteria pellet was sonicated and the resulting cell homogenate was incubated at room temperature for 15 days compensating the water evaporation by addition of distilled water. Then, different aliquots of the homogenates were diluted 1:1, 1:10, and 1:100 with distilled water and used to wet filter paper in petri dishes with three replicates with 10 plants for each concentration level. *Arabidopsis thaliana* seedlings were prepared and the experiment was performed as for Experiment 1.

### Results

A color change of the *A. platensis* culture after incubation with the *A. thaliana* DNA was observed, providing an indirect indication of the incorporation of *A. thaliana* DNA in the *A. platensis* cells. In fact, this observed color change indicates a change in gene expression, that is not unlikely to be the result of a change of the *A. platensis* genome due to incorporation of *A. thaliana* DNA.

The results of the inhibition tests demonstrated the inhibitory effects by mixtures of heterologous and self-DNA by testing cyanobacterial natural libraries of genomes of *Arabidopsis thaliana* on conspecific seedling survival. At high concentrations, seedling survival was significantly lower under exposure to the natural library of *A. platensis* which absorbed the *Arabidopsis* DNA compared to the control with the microalgae solution without *Arabidopsis* DNA (i.e. containing heterologous DNA but not the plant DNA) (Table 8).

Although, most probably only a fraction of the *A. thaliana* DNA is incorporated in the *A. platensis* genome (and thus replicated by the *A. platensis* cells), there is considerable inhibition of *A. thaliana* seedlings. In view of the results of the test on the inhibition by a fraction of the genome of C. *elegans* (see experiment 3), it may be expected that by combining the inhibitory transformed *A. platensis* culture with further cultures obtained with a similar transformation procedure, the inhibitory effects can be increased.

**Table 8. Inhibition of A. thaliana by transformed Arthrospira platensis**

| | **1:1** | **1:10** | **1:100** |
|---|---|---|---|
| **Control (*A*. platensis)** | 0% | 0% | 0% |
| ***A*. platensis NATURAL library** | 80% | 10% | 0% |

% of mortality (10 seedlings/treatment transplanted on petri dishes).

### EXPERIMENT 8

A confirmatory experiment was conducted to further confirm the inhibition of self-DNA incorporated in the natural genome of a host species. In this confirmatory experiment, two different species of cyanobacteria (photosynthetic microalgae) were grown in a bioreactor, fragmented Arabidopsis DNA was added to the growth medium and the inhibition of disrupted biomass from the cyanobacteria was tested.

The cyanobacteria selected were *Arthrospira platensis* (as in example 7) and *Synechocystis* sp. PCC6803. Before the incubation all cultures are maintained at the cultivation condition indicated in table 1 without insufflation at least from 4 hours before the incubation.

**Table 9 Species incubated and the corresponding cultivation conditions (Stein)**

| **Species incubated** | **Temperature (°C)** | **L:D (h:h)** | **Irradiance (umoles photons m⁻² sec ⁻¹)** | **Growth Medium** |
|---|---|---|---|---|
| *Arthrospira platensis* | 30 | 12:12 | 20 | Zarrouk's Medium |
| *Synechocystis* sp. PCC6803 | 25 | 12:12 | 20 | BG11 |

DNA isolated for harvested cellsis random fragmented by sonication bursts. The samples have been sonicated in a VC 505 SONICS Ultrasonic processor with a microtips immersed in a 1,5 ml microtubes with cold water in the water bath for 30 minutes with 45sec/15 sec on/off cycles at 20% of the amplitude.

### Incubation Arthrospira platensis with Arabidopsis thaliana DNA

Before the incubation *A. platensis* is cultivated in a Mg++ deficient growth medium in order to decrease the intracellular and extracellular DNAse activity of the cyanobacteria and to facilitate the persistence and incorporation of the foreign DNA (Cao et al 1999) During the incubation 300 ul of concentrated *A. platensis* cells ( up to 5* 107 cells obtained by gentle centrifugation) is added to a 15 ml centrifuge tube containing 300 mg of glass beads (500 µm of diameter) as described in Dunahay et al (1997). Then, in the incubation tube is added 100 ul of A. thaliana DNA previously fragmented by sonication bursts (about 10 ug) and 100 ul of Mg++ deficient medium and the tube is agitated using a vortex mixer at top speed for 15 seconds.

The sample volume is increased to 2,5 ml with Zarrouk's Mg++ deficient medium in the same tube and incubated in an oscillator under LD illumination cycles for 24 hours. Then the volume is progressively increased with the complete Zarrouk's Medium.

### Incubation Synechocystis sp. PCC6803 with Arabidopsis thaliana DNA

Before the incubation the cells has been cultured in BG11 medium modified with EDTA (2 mM) for two days in order to decrease the Ca++ dependent DNAse activity as described by Zang et al (2007).

During the incubation 400 ul of concentrated *Synechocystis* sp. PCC6803 cells ( up to 5* 107 cells obtained by gentle centrifugation and washed with BG11 EDTA free medium) is added to a 15 ml centrifuge tube containing 100 ul of *A. thaliana* DNA previously fragmented by sonication bursts (about 10 ug). The tube is then incubated in an oscillator under LD illumination cycles for 5 hours and then transferred to a flask where it is diluted to 2,5 ml with BG11 medium and the volume is then progressively increased.

### Experiment

The microalgae exposed to *Arabidopsis* DNA according to the above protocols were used as inoculum in two 5 liters bioreactors and grown in standard growth conditions until the reach of maximal cell density. Then, the algal biomass of the two species was extracted from the medium to form a pellet, carefully washed and then dried.

The algal pellets were inoculated with 1g of forest soil and maintained at room temperature and wet every week to keep natural decomposition for up to 60 days. The decaying material was sampled every 15 days and added on filter paper in lab optimal conditions in petri dishes to assess germination and root growth of Arabidopsis plants on plates.

### Results

Undecomposed pellets had no significant inhibitory effect on germination of seeds and root elongation of Arabidopsis, while after 30 and 60 days of decomposition a significant inhibition of germination was observed in treatment with decomposed pellets from both species. After 90 days of decomposition, the inhibitory effects of the decomposed pellets showed a slight decrease. This shows that the decomposition of the pellet of cyanobacteria has a clear inhibitory effect on the germination of plants as the DNA of the plant, included in the algal genome, is released by the decomposition process and eventually further amplified by the decomposing soil microorganisms.

**Table 10 - Results of inhibition test on Arabidopsis thaliana by exposure to (decomposed) biomass of two Arthrospira platensis (LEFT) and Synechocystis sp. PCC6803 (RIGHT).**

| **Treatment** | Germination (% control) | Root elongation (% control) |
|---|---|---|
| Control (water + fertilizer nutrients) | 100 / 100 | 100 / 100 |
| Undecomposed biomass | 100 / 95 | 95 / 90 |
| Decomposition 30 days | 80 / 65 | 75 / 60 |
| Decomposition 60 days | 50 / 30 | 50 / 20 |
| Decomposition 90 days | 55 / 40 | 50 / 25 |

### Bioinformatic analysis

The genome of the two cyanobacteria used as natural libraries in the abovementioned seed germination experiments was sequenced and analyzed. Paired-end reads from metagenomics experiments are merged using the BBmerged function of BBmap (Bushnell (2016)), applying the kmers option to reduce false positives (extend2=20, iterations=5).

Merged reads are then aligned versus the host genome using BLASTn (Camacho et al. (2009)). All the reads that have at least one hit where lister listed as total reads having a match and they are listed in groups according to their coverage. Possible recombinant fragments are those matching from 20 to 80 or 90 % coverage. Filtering at 90% identity is also considered. Details on the bioinformatic procedure can be found in Bushnell (2016) and Camacho et al, (2009).

**Table 11. Results of bioinformatics analysis**

| **Matching class** | **Class description** | **Arthrospira platensis** | **Synechocystis sp. PCC 6803** |
|---|---|---|---|
| 1 | 100% coverage with both A. thaliana and cyanobacterial genomes | 16 | 82 |
| 2 | 100% coverage with A. thaliana genome, partial coverage (between 10% and 99%) with cyanobacterial genome | 14 | 5 |
| 3 | Partial (between 10% and 99%) coverage with A. thaliana genome, 100% coverage with cyanobacterial genome | 498 | 187542 |
| 4 | Partial (between 10% and 99%) coverage with both A. thaliana and cyanobacterial genomes | 3524 | 5881 |
| 5 | 100% coverage with A. thaliana genome, no match with cyanobacterial genome | 0 | 0 |
| **All** | **Coverage > 10% with A. thaliana genome, any coverage with cyanobacterial genome** | **4052** | **193510** |

Table 11 shows that the counts of sequences, found in DNA from cyanobacterial samples, that matched *A. thaliana* genome largely varied not only between the samples, but also among different matching classes defined according to the percent coverage with the plant and the cyanobacterial genomes.

Classes 1 and 2 showed very low counts, possibly related to the uncertainty intrinsic of sequencing and bioinformatics methods. Class 3 showed the highest between-samples difference, possibly related to differences in genome size, chromosome copy numbers and data availability for blasting in reference genomic databases between the two cyanobacteria. Class 4 data indicates the occurrence of possible chimeric sequences partially matching both the plant and the cyanobacterium genomes in both sample types. Class 5 showed null values, indicating that both sample types did not include free A. thaliana DNA, out of the chimeric sequences.

Remarkably, the *Synechocystis* sp. PCC 6803 sample that showed larger inhibitory effect on *A. thaliana* seedlings compared to the *Arthrospira platensis* sample, also showed a higher number of DNA sequences matching *A. thaliana genome,* both when considering sequence counts for all classes pooled, or separately for each meaningful class. This confirms that inhibitory effects are connected to self-DNA fragments and that these inhibitory effects of the self-DNA fragments are maintained when in association with host species DNA. On the basis of this experiment it is further confirmed that cells of a host-species can effectively replicate inhibitory self-DNA of a phylogenetically distant target species in their natural genome.

### EXPERIMENT 9

A further confirmatory experiment was performed by testing the inhibition of the weed *Portulaca oleracea* by fragmented self-DNA subjected to decomposition and absorption by a soil microbiome. The test was done in test plots of spinach cultivation on a farm where *Portulaca* infestation was a recurring problem.

A BAC library of *Portulaca oleracea* was first generated by Bio S&T (Montreal, Canada) with a methodology similar to the one used for the *Cyperus aesculentus* library discussed in experiment 2. An overnight culture of the library (Terrific Broth (TB) as culture medium, 250 rpm agitation, T= 37 °C) was used as
a starter culture for a bioreactor fermentation (1% of the useful volume of the bioreactor) of 60 L with a useful volume of 40 Litres.

As substrate for large-scale liquid fermentation the Nutrient Broth (NB) was used. Furthermore, 0.1% L-Arabinose was added to the total volume of the culture medium to induce the pSmart-Hindlll plasmid. The liquid fermentation was carried out for 2 days under controlled conditions of temperature, agitation, aeration, pH in order to obtain a fermentation broth with high cell density and which stimulated the production of plasmid DNA. The concentration achieved was about 5.6 x 10⁸ cell/ml as estimated by the OD₆₀₀.

### Inoculation and maturation of the compost

The fermentation broth obtained as previously described was subjected to boiling for about 15 minutes and subsequently added to peat at three different concentrations:
1. 30mL/kg (1)
2. 3mL/kg (dilution 1: 10)
3. 0.3mL/kg (dilution 1: 100)

The inoculated soil was left to mature in the air for about 20 days. The product thus obtained was used in field trials.

### Field trial

The field test was performed at a farm in the Salerno (IT) region having recurring problems with *Portulaca* infestations.

Two days before the spinach sowing (mid-July) the product was distributed according to the following scheme:
Field experiment (dimension block around 2x2.4m)

| | | | | | |
|---|---|---|---|---|---|
| **CNT** | **1** | **CNT** | **1:10** | **CNT** | **1:100** |
| **1:100** | **CNT** | **1:10** | **CNT** | **1** | **CNT** |

| | | | | | |
|---|---|---|---|---|---|
| (CNT = Control (peat without addition of boiled fermentation broth)) | | | | | |

In the tunnels adjacent to the one prepared for the experimental tests, the fight against the *Portulaca* weed was carried out with conventional agronomic practices and the use of herbicides admitted according to the current legislation, constituting a further control, in order to evaluate the efficacy of the product in exam.

In particular, the usual practice of struggle adopted by the farmer provides for the use of Metam-sodium that is an agricultural general use pesticide used primarily as a broadspectrum pre-plant soil fumigant to control weeds, weed seeds, fungi, nematodes, and soil insects. Treatments with Metam-sodium are expensive and detrimental for the soil causing a loss of rhizosphere biodiversity.

At the spinach sprouting (about 10 days after sowing) the first survey was carried out, from here on the other evaluations were done on a weekly basis until the end of the life cycle of cultivated spinach. In order to ascertain the inhibitory effect of the product on the *Portulaca* germination rate, during the filed evaluation *Portulaca* plants were eradicated, counted and the roots were checked for the presence of necrosis.

### Results

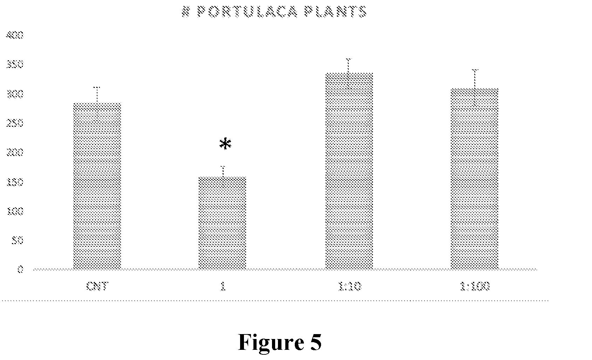

As is shown in Fig. 5 there was a significant reduction of the number of *Portulaca* plants in the block treatment 1 as determined at the end of the survey. Furthermore, as expected, in the block with less *Portulaca* plants the number of germinated spinach was significantly higher.

This experiment further confirms that inhibitory effects are connected to self-DNA fragments and that these inhibitory effects of the self-DNA fragments are maintained when the self-DNA fragments are amplified in a host species and are in association with host species DNA. In view of the experimental protocol used and in consideration of the results of the experiments above, it is reasonable to expect that amplification of the *Portulaca* DNA fragments by the peat microbiome must have contributed to the inhibitory effect.

### EXPERIMENT 10

### Inhibition by DNA fragments from phylogenetically similar species

Inhibition by DNA fragments from phylogenetically similar species was tested with fragmented DNA according to WO2014/020624 (not associated with host species DNA sequences). In view of the results from the other experiments, it may be expected that where inhibitory effects are observed for DNA fragments according to WO2014/020624, effects will also be observed for DNA in the composition according to the present invention, when fragmented.

### Extraction and quantification of DNA from plant materials

DNA was extracted from plant leaf materials by using a DNeasy Plant Maxi Kit as described by the manufacturer (QIAGEN, Valencia, CA) to prepare the treatment solution using the protocol by Fulton *et al.,* (1995) (Annex E). 100 grams fresh weight of starting material were harvested and frozen at -20°C. Then, the samples were blended immersed in 200 ml fresh microprep buffer, then filtered in cheese cloth. DNA was resuspended in pure sterile water. The extracted DNA from leaves was fragmented by sonication to a target size range of 200-500 bp. This was performed by a Bandelin Sonopulse (Bandelin, Berlin, DE) at 90% power with a 0.9 seconds pulse for 12 minutes. Verification of sonicated bands size was performed visually verified on 3% MetaPhor^{™} agarose gel (Lonza scientific, NJ) by Sybr^{®} Safe (Invitrogen).

### Bioassay with purified DNA on target plants

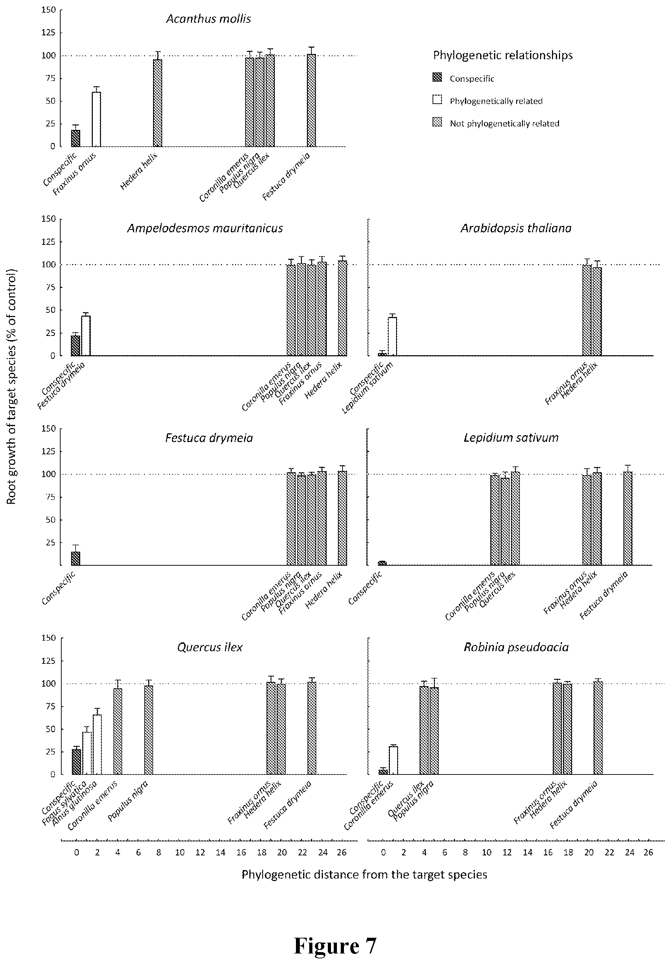

Autotoxic and phytotoxic effects of purified DNA extracted from plant leaves were assessed on eight target species (Figure 7). The experimental design was the following: 1. control with sterile distilled water; 2. addition of total extracted and randomly fragmented self-DNA; 3. addition of total extracted and randomly fragmented DNA from single heterospecifics (Table 1); DNA was applied at 200 µg ml⁻¹. Bioassays were done *in vitro* by using surface sterile seeds (n=20 in each plate) for each target species (4 replicates) placed in 9 cm Petri dishes over sterile filter papers imbibed with 4 ml of test solutions. Seedling root length was measured after a variable number of days since experiment start depending on the target species (Figure 7). Petri dishes were arranged in a growth chamber (temperature 22±2°C, watered every two days with distilled water), according to a totally randomized design.

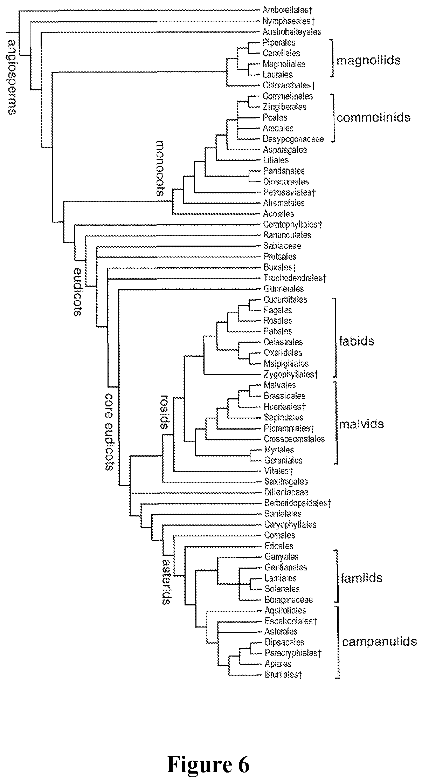

For each bioassay, the phylogenetic distance separating the target species and the plant species used as source for DNA extraction was assessed in the following way:
- conspecifcs at zero distance
- species belonging to the same family at distance = 1
- species belonging to different families within the same order at distance = 2
- species from different orders at distance = 2 + N, with N equal to the number of tree nodes separating plant orders in a reference phylogenetic tree (APG 2009, Fig. 6) that reports the phylogeny of taxonomic orders of Angiosperms (i.e. flowering plants).

The species, family and order of all the plants used in the bioassays, as well as the resulting phylogenetic distances for all pairs of target and DNA source plant, are reported in figure 7.

Bioassay results (i.e. root growth of the target species) were expressed as percentage of the control treated with distilled water, and plotted as a function of the phylogenetic distance separating the DNA source from the target species.

### Results

The bioassay results show a clear inhibitory effect on root growth of the target species by their own DNA (Fig. 8). For DNA from species phylogenetically similar (within the same taxonomic order) to the target species, when tested, also considerable inhibitory effects were observed, although less that the effect of the DNA from the target species itself. Effects by heterologous DNA from species distant from the target species were insignificant.

### REFERENCES

Bergomaz, R., Boppré, M., 1986. A simple insect diet for rearing Arctiidae and other moths. *Journal of the Lepidopterists' Society,* 40, 131-137.
Bonanomi G, Incerti G, Barile E, Capodilupo M, Antignani V, Mingo A, Lanzotti V, Scala F, Mazzoleni S. 2011. Phytotoxicity, not nitrogen immobilization, explains plant litter inhibitory effects: evidence from solid-state 13C NMR spectroscopy. New Phytologist, 191: 1018-1030.
Bushnell B, 2016. BBMap short read aligner. University of California, Berkeley, California. URL http://sourceforge. net/projects/bbmap.
Camacho C, Coulouris G, Avagyan V, Ma N, Papadopoulos J, Bealer K, Madden TL. 2009. BLAST+: architecture and applications. BMC bioinformatics, 10(1), 421.
Cao, J., Xu, Z., Qiu, G. and Li, B., 1999. Effects of Mg2+ on the growth and DNase activity of Spirulina platensis, a cyanobacterium. Bioresource technology, 67(3), pp.287-290.
Dunahay, Terri G., Sally A. Adler, and Jonathan W. Jarvik. "Transformation of microalgae using silicon carbide whiskers." Recombinant Gene Expression Protocols. Humana Press, 1997. 503-509.
Mazzoleni, S., Bonanomi, G., Incerti, G., Chiusano, M.L., Termolino, P., Mingo, A., Senatore, M., Giannino, F., Carteni, F., Rietkerk, M. and Lanzotti, V., 2015a. Inhibitory and toxic effects of extracellular self-DNA in litter: a mechanism for negative plant-soil feedbacks? New Phytologist, 205(3), pp. 1195-1210.
Mazzoleni, S., Carteni, F., Bonanomi, G., Senatore, M., Termolino, P., Giannino, F., Incerti, G., Rietkerk, M., Lanzotti, V. and Chiusano, M.L., 2015b. Inhibitory effects of extracellular self-DNA: a general biological process? New Phytologist, 206(1), pp.127-132.
Smit AF, Hubley R, Green P. 1996. RepeatMasker. URL: http://www.repeatmasker.org.
Sang JH (1978) The nutritional requirements of Drosophila. The Genetics and Biology of Drosophila,Vol. 2 (ed. By M. Ashburner & TRF Wright), pp. 159-192. Academic Press, New York, NY, USA.
Stein, J. (ED.) Handbook of Phycological methods. Culture methods and growth measurements. Cambridge University Press. 448 pp.
Zang, X., Liu, B., Liu, S., Arunakumara, K. K. I. U., & Zhang, X. (2007). Optimum conditions for transformation of Synechocystis sp. PCC 6803. The Journal of Microbiology, 45(3), 241-245.
Angiosperm Phylogeny Group (AGP), 2009. An update of the Angiosperm Phylogeny Group classification for the orders and families of flowering plants. Botanical Journal of the Linnean Society 161(2 ): 105-121, DOI:10.1111/j.1095-8339.2009.00996.x.
Fulton TM, Chunwongse J, Tanksley SD. 1995. Microprep protocol for extraction of DNA from tomato and other herbaceous plants. Plant Molecular Biology Reporter 13: 207-209.

## Claims

1. Non-medical use of a composition as a product for inhibiting a species, the target species, said composition comprising a mixture of DNA molecules, having DNA sequences from different sources, said mixture comprising a number of first DNA sequences, preferably from chromosomal DNA sequences, of a species, the source species, and a number of second DNA sequences, preferably chromosomal DNA sequences, from a species, the host species, used to replicate the source species DNA, said host species differing from the source species, wherein the source species is selected from the target species or is a species phylogenetically similar to the target species, wherein species are phylogenetically similar if they are from the same family, preferably from the same genus, and wherein the source species and the host species are phylogenetically distant by being selected from different taxonomic orders, such as by being selected from different classes, different phyla, different kingdoms, or different domains.

2. Use according to claim 1, wherein the mixture of DNA molecules comprises chimeric DNA molecules wherein source species DNA sequences are flanked on at least one end by non-source species DNA sequences, said non-source species DNA sequences being selected from host species DNA sequences, preferably chromosomal DNA sequences from the natural genome of the host species, or from DNA sequences from an artificial DNA construct, such as selected from a plasmid, a cosmid or an artificial chromosome.

3. Use according to claims 1-2, wherein the composition comprises a population of cells of the host species, preferably from a host species selected from a microbial species, wherein the DNA molecules are present in host species cells, preferably in viable host species cells.

4. Use according to any of the claims 1-3, wherein the DNA molecules comprise unfragmented chimeric DNA molecules and the source species DNA sequences comprise partial DNA sequences, preferably randomly generated partial DNA sequences of chromosomal DNA, or comprise cDNA sequences reverse transcribed from a transcriptome of the host species, incorporated in the chimeric DNA molecules.

5. Use according to claims 1-2, wherein the DNA molecules are fragmented DNA molecules.

6. Use according to any of the claims 1-5, wherein non-source species DNA, preferably host species DNA, is in excess of source species DNA, preferably such that the ratio of non-source species DNA : source species DNA is between 10:1 and 1000: 1, such as between 20:1 and 500:1, 50:1 and 500:1, 100:1 and 500:1.

7. Use according to any of the claims 1-6, wherein the host species is selected from a microbial species, such as a bacterial species, or a species from the Ascomycota, or a species from the Archaea, or a microphyte, a multicellular organism, such as a multicellular plant, or a helminth species, a soil microorganism, a GRAS status microorganism, a microbial biocontrol agent.

8. Use according to claim 7, wherein a bacterial species is selected from, *E. coli, a* Bacillus species, a *Pseudomonas* species, lactic acid bacteria, such as *Lactobacillus* spp., *Leuconostoc* spp., *Pediococcus* spp., *Lactococcus* spp., or *Streptococcus* spp, a cyanobacteria species, such as an *Arthrospira* species, in particular *Arthrospira plantenis* or *Arthrospira maxima,* a microbial species from the Ascomycota is selected from a *Saccharomyces* species, such as *saccharomyces cerevisiae,* or an *Aspergillus* species, such as *Aspergillus niger, Aspergillus oryzae, or Aspergillus nidulans, a* multicellular plant is selected from a crop, preferably a food crop, such as selected from cereals, corn, sugar beet, rapeseed, pea, soybean, an oil crop, such as rapeseed, Ethiopian mustard, sunflower, a starch crop, such as (sweet) sorghum, a fibre crop, such as flax or hemp, a lignocellulosic crop, such as reed canary grass, giant reed, switchgrass, miscanthus, cardoon, a short rotation forestry crop, such as willow, poplar, or eucalyptus, a helminth species is selected from a helminth used in helminthic therapy such as *Trichuris suis, Necator americanus, Trichuris trichiura, Hymenolepis diminuta, Ascaris lumbricoides, Strongyloides stercoralis, Enterobius vermicularis* or *Hymenolepis nana,* a soil microorganism is selected from *Alphaproteobacteria* spp., *Betaproteobacteria* spp., *Deltaproteobacteria* spp., *Actinobacteria* spp., *Thermoleophilia* spp., *Rubrobacteria* spp., *Chloracidobacteria* spp., *Acidobacteria* spp., or *Solibacteres* spp., a microbial biocontrol agent, is selected from a bacterial biocontrol agent, in particular *Bacillus thuringiensis* or *Bacillus subtillus,* or a fungal biocontrol agent, for example an entomopathogenic fungi, such as *Beauveria bassiana, Isaria fumosorosea, Lecanicillium* spp., or *Metarhizium* spp. or for example *Trichoderma* spp. or *Ampelomyces quisqualis.*

9. Use according to any of the claims 1-8, wherein the target species is a disease associated species, such as a pathogenic species, a parasitic species or a species serving as a disease vector, or is an infesting species, or is a species associated with deterioration of products, such as of food products and/or of cosmetic products and/or of pharmaceutical products and/or of other products comprising organic matter.

10. Use according to claim 9, wherein the target species is selected from a pathogenic species, preferably a topical pathogenic target species, most preferably pathogenic target species selected from Archaea, bacteria, fungi (including yeasts) and protists, a parasitic species, in particular a species parasitic to humans and/or life stock and/or a pet animal, preferably a parasitic target species selected from skin parasites and/or gastrointestinal parasites and/or mucosal parasites, a phytopathogenic species, such as a plant pathogen selected from fungi, Oomycetes, bacteria, viruses or protists, an agricultural pest arthropod, a disease vector for an animal disease, preferably a disease vector selected from Arthropods, a disease vector for a plant disease, preferably a disease vector selected from Arthropods, a nematode species parasitic to plants, a weed species, or species that cause product deterioration.

11. Use according to any of the claims 1-10, wherein the host species comprises a food source for the target species, for example wherein the host species is selected from a bacterial species and the target species is selected from a bacteria-feeding nematode species, or wherein the host species is a multicellular plant and the target species is a phytophagous species, such as a phytophagous arthropod species or a terrestrial arthropod species.

12. Use according to any of the claims 1-11, wherein DNA of an artificial DNA construct selected from a plasmid, a cosmid, a fosmid, or an artificial chromosome, such as a bacterial artificial chromosome, a yeast artificial chromosome, or a fungus artificial chromosome, is present in the composition.

13. Use according to any of the claims 1-12, wherein the target species is selected from Lepidoptera, in particular selected from the family Tortricidae, such as from the genus *Choristoneura,* in particular *Choristoneura orae, Choristoneura fumiferana* or *Choristoneura freemani,* or selected from the family Noctuidae, such as the genus *Spodoptera,* in particular *Spodoptera frugiperda, Spodoptera litura, Spodoptera litoralis, Spodoptera cilium* or *Spodoptera ornithogalli,* or is selected from the family Pyralidae, such as from the genus *Plodia* or *Ephestia,* or from the Diptera, Coleoptera, or Hymenoptera, and most preferably is selected from the genus *Choristoneura,* and the composition further comprises a biocontrol agent directed against the target species, such as a bacterial biocontrol agent, for example *Bacillus thuringiensis* or *Bacillus subtillus,* or a fungal biocontrol agent, for example an entomopathogenic fungus, such as *Beauveria bassiana, Isaria fumosorosea, Lecanicillium* spp., or a *Metarhizium* spp, wherein the biocontrol agent preferably is *Bacillus thuringiensis* biomass, wherein most preferably *Bacillus thuringiensis* biomass is viable host species cells.

14. Composition for use as a medicamentfor the treatment of an infection with a target species selected from a parasite and/or pathogen, preferably an infection with a pathogen and/or parasite of skin, nail or a mucous membrane, wherein the composition is as defined in any of the claims 1-13 and is used as an inhibitory agent against the target species.

15. Non-therapeutic method of inhibiting a species, the target species, comprising exposing said target species to a composition as defined in any of the claims 1-13.

16. Method of producing a composition for use as an inhibiting agent against a target species, wherein the composition is as defined in claims 1-13 and the method comprises:
- providing a source of DNA from the source species, optionally comprising fragmented source species DNA;
- providing cells of a number of species, the host species, differing from the source species;
- subjecting the host species cells with the source of DNA from the source species to conditions allowing the host species cells to take up source species DNA in a replicable form;
- isolating host species cells as a source of DNA inhibitory to the target species;
- optionally, isolating DNA inhibitory to the target species from the host species cells;
- optionally, fragmenting the DNA content of host species cells.

## Patentansprüche

1. Nicht-medizinische Verwendung einer Zusammensetzung als Produkt zur Hemmung einer Spezies, der Zielspezies, wobei die Zusammensetzung eine Mischung von DNA-Molekülen umfasst, die DNA-Sequenzen aus verschiedenen Quellen aufweist, wobei die Mischung eine Anzahl erster DNA-Sequenzen, vorzugsweise aus chromosomalen DNA-Sequenzen, einer Spezies, der Quellspezies, und eine Anzahl zweiter DNA-Sequenzen, vorzugsweise chromosomaler DNA-Sequenzen, aus einer Spezies, der Wirtsspezies, umfasst, die zur Replikation der DNA der Quellspezies verwendet wird, wobei sich die Wirtsspezies von der Quellspezies unterscheidet, wobei die Quellspezies aus der Zielspezies ausgewählt ist oder eine Spezies ist, die der Zielspezies phylogenetisch ähnlich ist, wobei Spezies phylogenetisch ähnlich sind, wenn sie aus derselben Familie stammen, vorzugsweise aus derselben Gattung, und wobei die Quellspezies und die Wirtsspezies phylogenetisch entfernt sind, indem sie aus verschiedenen taxonomischen Ordnungen ausgewählt sind, etwa indem sie aus verschiedenen Klassen, verschiedenen Stämmen, verschiedenen Reichen oder verschiedenen Domänen ausgewählt sind.

2. Verwendung nach Anspruch 1, wobei die Mischung von DNA-Molekülen chimäre DNA-Moleküle umfasst, wobei DNA-Sequenzen der Quellspezies an mindestens einem Ende von DNA-Sequenzen einer Nicht-Quellspezies flankiert werden, wobei die genannten DNA-Sequenzen der Nicht-Quellspezies aus DNA-Sequenzen der Wirtsspezies, vorzugsweise chromosomalen DNA-Sequenzen aus dem natürlichen Genom der Wirtsspezies, oder aus DNA-Sequenzen aus einem künstlichen DNA-Konstrukt ausgewählt sind, etwa ausgewählt aus einem Plasmid, einem Cosmid oder einem künstlichen Chromosom.

3. Verwendung nach den Ansprüchen 1-2, wobei die Zusammensetzung eine Population von Zellen der Wirtsspezies umfasst, vorzugsweise aus einer Wirtsspezies, die aus einer mikrobiellen Spezies ausgewählt ist, wobei die DNA-Moleküle in Zellen der Wirtsspezies vorhanden sind, vorzugsweise in lebensfähigen Zellen der Wirtsspezies.

4. Verwendung nach einem der Ansprüche 1-3, wobei die DNA-Moleküle unfragmentierte chimäre DNA-Moleküle umfassen und die DNA-Sequenzen der Quellspezies partielle DNA-Sequenzen umfassen, vorzugsweise zufällig erzeugte partielle DNA-Sequenzen chromosomaler DNA, oder cDNA-Sequenzen umfassen, die von einem Transkriptom der Wirtsspezies revers transkribiert und in die chimären DNA-Moleküle eingebaut wurden.

5. Verwendung nach den Ansprüchen 1-2, wobei die DNA-Moleküle fragmentierte DNA-Moleküle sind.

6. Verwendung nach einem der Ansprüche 1-5, wobei DNA einer Nicht-Quellspezies, vorzugsweise DNA der Wirtsspezies, im Überschuss gegenüber DNA der Quellspezies vorliegt, vorzugsweise so, dass das Verhältnis von DNA der Nicht-Quellspezies zu DNA der Quellspezies zwischen 10:1 und 1000:1 liegt, zum Beispiel zwischen 20:1 und 500:1, 50:1 und 500:1, 100:1 und 500:1.

7. Verwendung nach einem der Ansprüche 1-6, wobei die Wirtsspezies aus einer mikrobiellen Spezies ausgewählt ist, etwa einer bakteriellen Spezies, oder einer Spezies aus den Ascomycota, oder einer Spezies aus den Archaea, oder einem Mikrophyten, einem mehrzelligen Organismus, etwa einer mehrzelligen Pflanze, oder einer Helminthenart, einem Bodenmikroorganismus, einem Mikroorganismus mit GRAS-Status, einem mikrobiellen Biocontrol-Mittel.

8. Verwendung nach Anspruch 7, wobei eine bakterielle Spezies ausgewählt ist aus *E. coli*, einer Bacillus-Spezies, einer *Pseudomonas*-Spezies, Milchsäurebakterien, etwa *Lactobacillus* spp., *Leuconostoc* spp., *Pediococcus* spp., *Lactococcus* spp. oder *Streptococcus* spp., einer Cyanobakterien-Spezies, etwa einer *Arthrospira*-Spezies, insbesondere *Arthrospira plantenis* oder *Arthrospira maxima*, eine mikrobielle Spezies aus den Ascomycota ausgewählt ist aus einer *Saccharomyces*-Spezies, etwa *Saccharomyces cerevisiae*, oder einer *Aspergillus*-Spezies, etwa *Aspergillus niger*, *Aspergillus oryzae* oder *Aspergillus nidulans*, eine mehrzellige Pflanze ausgewählt ist aus einer Nutzpflanze, vorzugsweise einer Nahrungspflanze, etwa ausgewählt aus Getreide, Mais, Zuckerrübe, Raps, Erbse, Sojabohne, einer Ölpflanze, etwa Raps, Äthiopischer Senf, Sonnenblume, einer Stärkepflanze, etwa (Zucker-)Sorghum, einer Faserpflanze, etwa Flachs oder Hanf, einer lignocellulosehaltigen Pflanze, etwa Rohrglanzgras, Riesenschilf, Rutenhirse, Miscanthus, Cardoon, einer Energieholzpflanze, etwa Weide, Pappel oder Eukalyptus, wobei eine Helminthenart ausgewählt ist aus einem in der Helminthentherapie verwendeten Helminthen, etwa *Trichuris suis, Necator americanus, Trichuris trichiura*, *Hymenolepis diminuta, Ascaris lumbricoides, Strongyloides stercoralis, Enterobius vermicularis* oder *Hymenolepis nana*, ein Bodenmikroorganismus ausgewählt ist aus *Alphaproteobacteria* spp., *Betaproteobacteria* spp., *Deltaproteobacteria* spp., *Actinobacteria* spp., *Thermoleophilia* spp., *Rubrobacteria* spp., *Chloracidobacteria* spp., *Acidobacteria* spp. oder *Solibacteres* spp., ein mikrobielles Biocontrol-Mittel ausgewählt ist aus einem bakteriellen Biocontrol-Mittel, insbesondere *Bacillus thuringiensis* oder *Bacillus subtillus*, oder einem pilzlichen Biocontrol-Mittel, beispielsweise einem entomopathogenen Pilz, etwa *Beauveria bassiana*, *Isaria fumosorosea*, *Lecanicillium* spp. oder *Metarhizium* spp. oder beispielsweise *Trichoderma* spp. oder *Ampelomyces quisqualis*.

9. Verwendung nach einem der Ansprüche 1-8, wobei die Zielspezies eine krankheitsassoziierte Spezies ist, etwa eine pathogene Spezies, eine parasitäre Spezies oder eine als Krankheitsvektor dienende Spezies, oder eine Schädlingsspezies ist, oder eine Spezies ist, die mit der Verschlechterung von Produkten assoziiert ist, etwa von Nahrungsmitteln und/oder Kosmetikprodukten und/oder pharmazeutischen Produkten und/oder anderen Produkten, die organisches Material umfassen.

10. Verwendung nach Anspruch 9, wobei die Zielspezies ausgewählt ist aus einer pathogenen Spezies, vorzugsweise einer topischen pathogenen Zielspezies, am meisten bevorzugt pathogenen Zielspezies, die aus Archaea, Bakterien, Pilzen (einschließlich Hefen) und Protisten ausgewählt sind, einer parasitären Spezies, insbesondere einer für Menschen und/oder Nutzvieh und/oder ein Haustier parasitären Spezies, vorzugsweise einer parasitären Zielspezies, die aus Hautparasiten und/oder Magen-Darm-Parasiten und/oder Schleimhautparasiten ausgewählt ist, einer phytopathogenen Spezies, etwa einem Pflanzenpathogen, das aus Pilzen, Oomyceten, Bakterien, Viren oder Protisten ausgewählt ist, einem landwirtschaftlichen Schädlingsarthropoden, einem Krankheitsvektor für eine Tierkrankheit, vorzugsweise einem aus Arthropoden ausgewählten Krankheitsvektor, einem Krankheitsvektor für eine Pflanzenkrankheit, vorzugsweise einem aus Arthropoden ausgewählten Krankheitsvektor, einer für Pflanzen parasitären Nematodenart, einer Unkrautart oder Spezies, die eine Produktverschlechterung verursachen.

11. Verwendung nach einem der Ansprüche 1-10, wobei die Wirtsspezies eine Nahrungsquelle für die Zielspezies umfasst, beispielsweise wobei die Wirtsspezies aus einer bakteriellen Spezies ausgewählt ist und die Zielspezies aus einer bakterienfressenden Nematodenart ausgewählt ist, oder wobei die Wirtsspezies eine mehrzellige Pflanze ist und die Zielspezies eine phytophage Spezies ist, etwa eine phytophage Arthropodenart oder eine terrestrische Arthropodenart.

12. Verwendung nach einem der Ansprüche 1-11, wobei DNA eines künstlichen DNA-Konstrukts, das aus einem Plasmid, einem Cosmid, einem Fosmid oder einem künstlichen Chromosom ausgewählt ist, etwa einem bakteriellen künstlichen Chromosom, einem künstlichen Hefechromosom oder einem künstlichen Pilzchromosom, in der Zusammensetzung vorhanden ist.

13. Verwendung nach einem der Ansprüche 1-12, wobei die Zielspezies aus Lepidoptera ausgewählt ist, insbesondere ausgewählt aus der Familie Tortricidae, etwa aus der Gattung *Choristoneura*, insbesondere *Choristoneura* orae, *Choristoneura fumiferana* oder *Choristoneura freemani*, oder ausgewählt ist aus der Familie Noctuidae, etwa der Gattung *Spodoptera*, insbesondere *Spodoptera frugiperda*, *Spodoptera litura*, *Spodoptera litoralis*, *Spodoptera cilium* oder *Spodoptera ornithogalli*, oder ausgewählt ist aus der Familie Pyralidae, etwa aus der Gattung *Plodia* oder *Ephestia*, oder aus den Diptera, Coleoptera oder Hymenoptera, und am meisten bevorzugt aus der Gattung *Choristoneura* ausgewählt ist, und die Zusammensetzung ferner ein gegen die Zielspezies gerichtetes Biocontrol-Mittel umfasst, etwa ein bakterielles Biocontrol-Mittel, beispielsweise *Bacillus thuringiensis* oder *Bacillus subtillus*, oder ein pilzliches Biocontrol-Mittel, beispielsweise einen entomopathogenen Pilz, etwa *Beauveria bassiana*, *Isaria fumosorosea*, *Lecanicillium* spp. oder ein *Metarhizium* spp., wobei das Biocontrol-Mittel vorzugsweise *Bacillus-thuringiensis-Biomasse* ist, wobei am meisten bevorzugt die *Bacillus-thuringiensis-Biomasse* lebensfähige Zellen der Wirtsspezies sind.

14. Zusammensetzung zur Verwendung als Medikament zur Behandlung einer Infektion mit einer aus einem Parasiten und/oder Pathogen ausgewählten Zielspezies, vorzugsweise einer Infektion mit einem Pathogen und/oder Parasiten der Haut, des Nagels oder einer Schleimhaut, wobei die Zusammensetzung wie in einem der Ansprüche 1-13 definiert ist und als hemmendes Mittel gegen die Zielspezies verwendet wird.

15. Nicht-therapeutisches Verfahren zur Hemmung einer Spezies, der Zielspezies, umfassend das Aussetzen der genannten Zielspezies gegenüber einer Zusammensetzung, wie in einem der Ansprüche 1-13 definiert.

16. Verfahren zur Herstellung einer Zusammensetzung zur Verwendung als hemmendes Mittel gegen eine Zielspezies, wobei die Zusammensetzung wie in den Ansprüchen 1-13 definiert ist und das Verfahren Folgendes umfasst:
- Bereitstellen einer DNA-Quelle aus der Quellspezies, gegebenenfalls umfassend fragmentierte DNA der Quellspezies;
- Bereitstellen von Zellen einer Anzahl von Spezies, der Wirtsspezies, die sich von der Quellspezies unterscheiden;
- Aussetzen der Zellen der Wirtsspezies mit der DNA-Quelle aus der Quellspezies gegenüber Bedingungen, die es den Zellen der Wirtsspezies erlauben, DNA der Quellspezies in einer replizierbaren Form aufzunehmen;
- Isolieren von Zellen der Wirtsspezies als Quelle von für die Zielspezies hemmender DNA;
- gegebenenfalls Isolieren von für die Zielspezies hemmender DNA aus den Zellen der Wirtsspezies;
- gegebenenfalls Fragmentieren des DNA-Gehalts der Zellen der Wirtsspezies.

## Revendications

1. Utilisation non médicale d'une composition en tant que produit destiné à inhiber une espèce, l'espèce cible, ladite composition comprenant un mélange de molécules d'ADN présentant des séquences d'ADN provenant de différentes sources, ledit mélange comprenant un ensemble de premières séquences d'ADN, de préférence, des séquences d'ADN chromosomique, provenant d'une espèce, l'espèce source, et un ensemble de secondes séquences d'ADN, de préférence des séquences d'ADN chromosomique, provenant d'une espèce, l'espèce hôte, utilisée pour répliquer l'ADN de l'espèce source, ladite espèce hôte étant différente de l'espèce source, dans lequel l'espèce source est choisie parmi l'espèce cible ou une espèce phylogénétiquement proche de l'espèce cible, les espèces étant considérées comme phylogénétiquement proches lorsqu'elles appartiennent à la même famille. de préférence du même genre, et dans lequel l'espèce source et l'espèce hôte sont phylogénétiquement éloignées, en ce qu'elles sont choisies parmi des ordres taxonomiques différents, par exemple parmi des classes différentes, des embranchements différents, des règnes différents ou des domaines différents.

2. Utilisation selon la revendication 1, dans laquelle le mélange de molécules d'ADN comprend des molécules d'ADN chimériques, dans lesquelles les séquences d'ADN de l'espèce source sont flanquées, à, au moins une extrémité, de séquences d'ADN ne provenant pas de l'espèce source, lesdites séquences d'ADN ne provenant pas de l'espèce source étant choisies parmi des séquences d'ADN de l'espèce hôte, de préférence des séquences d'ADN chromosomique issues du génome naturel de l'espèce hôte, ou parmi des séquences d'ADN provenant d'une construction d'ADN artificielle, notamment choisies parmi un plasmide, un cosmide ou un chromosome artificiel.

3. Utilisation selon les revendications 1 à 2, dans laquelle la composition comprend une population de cellules de l'espèce hôte, de préférence d'une espèce hôte choisie parmi les espèces microbiennes, les molécules d'ADN étant présentes dans les cellules de l'espèce hôte, de préférence dans des cellules viables de l'espèce hôte.

4. Utilisation selon l'une quelconque des revendications 1 à 3, dans laquelle les molécules d'ADN comprennent des molécules d'ADN chimériques non fragmentées et les séquences d'ADN de l'espèce source comprennent des séquences d'ADN partielles, de préférence des séquences partielles d'ADN chromosomique générées aléatoirement, ou comprennent des séquences d'ADNc obtenues par transcription inverse à partir du transcriptome de l'espèce hôte et incorporées dans les molécules d'ADN chimériques.

5. Utilisation selon les revendications 1 à 2, dans laquelle les molécules d'ADN sont des molécules d'ADN fragmentées.

6. Utilisation selon l'une quelconque des revendications 1 à 5, dans laquelle l'ADN ne provenant pas de l'espèce source, de préférence l'ADN de l'espèce hôte, est présent en excès par rapport à l'ADN de l'espèce source, de préférence de telle sorte que le rapport ADN ne provenant pas de l'espèce source : ADN de l'espèce source est compris entre 10:1 et 1000:1, notamment entre 20:1 et 500:1, entre 50:1 et 500:1 ou entre 100:1 et 500:1.

7. Utilisation selon l'une quelconque des revendications 1 à 6, dans laquelle l'espèce hôte est choisie parmi une espèce microbienne, telle qu'une espèce bactérienne, une espèce appartenant au phylum des Ascomycota, une espèce appartenant au domaine des Archaea, ou encore un microphyte, un organisme pluricellulaire, notamment une plante pluricellulaire, une espèce d'helminthe, un microorganisme du sol, un microorganisme bénéficiant du statut GRAS ou un agent microbien de lutte biologique.

8. Utilisation selon la revendication 7, dans laquelle l'espèce bactérienne est choisie parmi E. coli, une espèce du genre Bacillus, une espèce du genre Pseudomonas, les bactéries lactiques, telles que Lactobacillus spp., Leuconostoc spp., Pediococcus spp., Lactococcus spp. ou Streptococcus spp., une espèce de cyanobactéries, telle qu'une espèce du genre Arthrospira, en particulier Arthrospira platensis ou Arthrospira maxima, une espèce microbienne appartenant au phylum des Ascomycota est choisie parmi une espèce du genre Saccharomyces, telle que Saccharomyces cerevisiae, ou une espèce du genre Aspergillus, telle que Aspergillus niger, Aspergillus oryzae ou Aspergillus nidulans, une plante pluricellulaire est choisie parmi une culture agricole, de préférence une culture alimentaire, notamment parmi les céréales, le maïs, la betterave sucrière, le colza, le pois ou le soja, une culture oléagineuse, telle que le colza, la moutarde d'Éthiopie ou le tournesol, une culture amylacée, telle que le sorgho (y compris le sorgho sucré), une culture à fibres, telle que le lin ou le chanvre, une culture lignocellulosique, telle que l'alpiste roseau, le roseau géant, le panic érigé, le miscanthus ou le cardon, une culture forestière à rotation courte, telle que le saule, le peuplier ou l'eucalyptus, une espèce d'helminthe est choisie parmi les helminthes utilisés en thérapie helminthique, tels que Trichuris suis, Necator americanus, Trichuris trichiura, Hymenolepis diminuta, Ascaris lumbricoides, Strongyloides stercoralis, Enterobius vermicularis ou Hymenolepis nana ; un microorganisme du sol est choisi parmi Alphaproteobacteria spp., Betaproteobacteria spp., Deltaproteobacteria spp., Actinobacteria spp., Thermoleophilia spp., Rubrobacteria spp., Chloracidobacteria spp., Acidobacteria spp. ou Solibacteres spp., et un agent microbien de lutte biologique est choisi parmi un agent bactérien de lutte biologique, en particulier Bacillus thuringiensis ou Bacillus subtilis, ou un agent fongique de lutte biologique, par exemple un champignon entomopathogène, tel que Beauveria bassiana, Isaria fumosorosea, Lecanicillium spp. ou Metarhizium spp. ou par exemple Trichoderma spp. ou Ampelomyces quisqualis.

9. Utilisation selon l'une quelconque des revendications 1 à 8, dans laquelle l'espèce cible est une espèce associée à une maladie, telle qu'une espèce pathogène, une espèce parasite ou une espèce servant de vecteur de maladie, ou est une espèce nuisible, ou est une espèce associée à la détérioration de produits, tels que des produits alimentaires et/ou des produits cosmétiques et/ou des produits pharmaceutiques et/ou d'autres produits comprenant de la matière organique.

10. Utilisation selon la revendication 9, dans laquelle l'espèce cible est choisie parmi une espèce pathogène, de préférence une espèce cible pathogène topique, plus préférablement une espèce cible pathogène choisie parmi les Archées, les bactéries, les champignons (y compris les levures) et les protistes, une espèce parasite, notamment une espèce parasite de l'être humain et/ou du bétail et/ou d'un animal de compagnie, de préférence une espèce cible parasite choisie parmi les parasites cutanés et/ou les parasites gastro-intestinaux et/ou les parasites des muqueuses, une espèce phytopathogène, telle qu'un agent pathogène des plantes choisi parmi les champignons, les Oomycètes, les bactéries, les virus ou les protistes, un arthropode ravageur agricole, un vecteur de maladie animale, de préférence un vecteur de maladie choisi parmi les Arthropodes, un vecteur de maladie végétale, de préférence un vecteur de maladie choisi parmi les Arthropodes, une espèce de nématode parasite des plantes, une espèce de plante adventice, ou une espèce provoquant la détérioration de produits.

11. Utilisation selon l'une quelconque des revendications 1 à 10, dans laquelle l'espèce hôte comprend une source alimentaire pour l'espèce cible, par exemple dans laquelle l'espèce hôte est choisie parmi une espèce bactérienne et l'espèce cible est choisie parmi une espèce de nématode se nourrissant de bactéries, ou dans laquelle l'espèce hôte est une plante pluricellulaire et l'espèce cible est une espèce phytophage, telle qu'une espèce d'arthropode phytophage ou une espèce d'arthropode terrestre.

12. Utilisation selon l'une quelconque des revendications 1 à 11, dans laquelle de l'ADN provenant d'une construction d'ADN artificielle choisie parmi un plasmide, un cosmide, un fosmide ou un chromosome artificiel, tel qu'un chromosome artificiel bactérien, un chromosome artificiel de levure ou un chromosome artificiel de champignon, est présent dans la composition.

13. Utilisation selon l'une quelconque des revendications 1 à 12, dans laquelle l'espèce cible est choisie parmi les Lépidoptères, notamment parmi la famille des Tortricidae, tels que le genre Choristoneura, en particulier Choristoneura orae, Choristoneura fumiferana ou Choristoneura freemani, ou est choisie parmi la famille des Noctuidae, telle que le genre Spodoptera, en particulier Spodoptera frugiperda, Spodoptera litura, Spodoptera litoralis, Spodoptera cilium ou Spodoptera ornithogalli, ou est choisie parmi la famille des Pyralidae, telle que le genre Plodia ou Ephestia, ou parmi les Diptères, les Coléoptères ou les Hyménoptères, et de manière particulièrement préférée est choisie parmi le genre Choristoneura, et la composition comprend en outre un agent de lutte biologique dirigé contre l'espèce cible, tel qu'un agent bactérien de lutte biologique, par exemple Bacillus thuringiensis ou Bacillus subtilis, ou un agent fongique de lutte biologique, par exemple un champignon entomopathogène, tel que Beauveria bassiana, Isaria fumosorosea, Lecanicillium spp. ou Metarhizium spp., dans laquelle l'agent de lutte biologique est, de préférence, une biomasse de Bacillus thuringiensis, et dans laquelle, de manière particulièrement préférée, la biomasse de Bacillus thuringiensis est constituée de cellules viables de l'espèce hôte.

14. Composition destinée à être utilisée comme médicament pour le traitement d'une infection par une espèce cible choisie parmi un parasite et/ou un agent pathogène, de préférence une infection par un agent pathogène et/ou un parasite de la peau, des ongles ou d'une muqueuse, dans laquelle la composition est telle que définie dans l'une quelconque des revendications 1 à 13 et est utilisée comme agent inhibiteur contre l'espèce cible.

15. Procédé non thérapeutique d'inhibition d'une espèce, l'espèce cible, comprenant l'exposition de ladite espèce cible à une composition telle que définie dans l'une quelconque des revendications 1 à 13.

16. Procédé de production d'une composition destinée à être utilisée comme agent inhibiteur contre une espèce cible, dans lequel la composition est telle que définie dans les revendications 1 à 13 et dans lequel le procédé comprend :
- la fourniture d'une source d'ADN provenant de l'espèce source, comprenant éventuellement de l'ADN fragmenté de l'espèce source ;
- la fourniture de cellules d'un ensemble d'espèces, les espèces hôtes, différentes de l'espèce source ;
- la soumission des cellules de l'espèce hôte à la source d'ADN provenant de l'espèce source dans des conditions permettant aux cellules de l'espèce hôte d'incorporer l'ADN de l'espèce source sous une forme réplicable ;
- l'isolement de cellules de l'espèce hôte comme source d'ADN inhibiteur de l'espèce cible ;
- éventuellement, l'isolement de l'ADN inhibiteur de l'espèce cible à partir des cellules de l'espèce hôte ;
- éventuellement, la fragmentation du contenu en ADN des cellules de l'espèce hôte.
